**Europäisches Patentamt**

(19) **European Patent Office**

**Office européen des brevets**

(11) Veröffentlichungsnummer: **0 030 353**
**B1**

(12) # EUROPÄISCHE PATENTSCHRIFT

(45) Veröffentlichungstag der Patentschrift:
**11.07.84**

(21) Anmeldenummer: **80107528.4**

(22) Anmeldetag: **02.12.80**

(51) Int. Cl.³: **A 61 M 5/32**

(54) **Verfahren und Vorrichtung zum Herstellen von Einmalkanülen.**

(30) Priorität: **10.12.79 DE 2949553**

(43) Veröffentlichungstag der Anmeldung:
**17.06.81 Patentblatt 81/24**

(45) Bekanntmachung des Hinweises auf die Patenterteilung:
**11.07.84 Patentblatt 84/28**

(84) Benannte Vertragsstaaten:
**AT BE CH DE FR GB IT LI LU NL SE**

(56) Entgegenhaltungen:
**CH - A - 394 500**
**DE - A - 846 769**
**DE - A - 1 295 140**
**DE - A - 1 491 725**
**DE - A - 1 491 743**
**DE - A - 1 704 102**
**FR - A - 1 357 757**
**US - A - 3 174 890**
**US - A - 3 436 803**
**US - A - 3 437 788**

(73) Patentinhaber: **SORTIMAT Creuz & Co. GmbH,
Postfach 250, D-7057 Winnenden (Württ.) (DE)**

(72) Erfinder: **Holzwarth, Friedrich, Espenweg 10,
D-7060 Schorndorf-Oberkerken (DE)**
Erfinder: **Heinze, Gerhard, Kernerstrasse 5,
D-7153 Weissach I.T. (DE)**

(74) Vertreter: **Dr. Elisabeth Jung Dr. Jürgen Schirdewahn Dr.
Gerhard Schmitt-Nilson Dr. Gerhard B. Hagen Dipl.-Ing.
Peter Hirsch, P.O. Box 40 14 68, D-8000 München 40 (DE)**

### Beschreibung

Die Herstellung von Spritzen, insbesondere für medizinische, aber auch für andere Zwecke, konzentriert sich zunehmend auf sog. Einwegspritzen, die nach einmaligem Gebrauch oder allenfalls nach einer relativ kleinen Folge von Benutzungen nicht mehr weiterverwendet werden. Derartige Spritzen müssen voll funktionsfähig sein, dürfen auf der anderen Seite aber nicht aufwendig und teuer wegen ihres überwiegend nur einmaligen Gebrauchs gestaltet sein. Es hat sich eingebürgert, für diesen Zweck sog. Einmalkanülen zu verwenden, bei denen ein metallisches Kanülenrohr mit einem Ansatz aus Kunststoff dicht und mechanisch fest verbunden wird. Mit dem Ansatz aus Kunststoff wird dann der Spritzenzylinder, welcher den Spritzenkolben enthält, durch Aufstecken oder fest, z.B. durch Kunststoffschweissung unter Wärmeeinwirkung, verbunden. Die Erfindung befasst sich mit der Herstellung derartiger Einmalkanülen aus einem metallischen Kanülenrohr mit einem Ansatz aus Kunststoff.

Bei allen solchen Einmalkanülen aus metallischem Kanülenrohr mit Kunststoffansatz ist die zugfeste Verbindung zwischen Kanülenrohr und Kunststoffansatz ein unabdingbares Erfordernis.

Es ist bereits vorgekommen, dass bei nicht hinreichend fester Verbindung der Unterdruck in einer Vene eines Patienten das Kanülenrohr mit erheblichem Impuls aus der Spritze in die Vene eingeschossen hat. Je nachdem, wo das Kanülenrohr dann im menschlichen Körper schliesslich seine endgültige Ruhelage findet, muss dann eine riskante und aufwendige Operation durchgeführt werden, ganz abgesehen davon, dass auch das eingeschossene Kanülenrohr selbst zu erheblichen Organbeschädigungen führen kann. Anderseits können Undichtigkeiten einmal zu einem Verlust von angesaugtem Fluid oder anderseits zu einer Emboliegefährdung durch eingedrungene Luft führen.

Neben der Zugfestigkeit soll das Kanülenrohr aber auch Druckfestigkeit gegen Hineindrücken in den Ansatz besitzen.

Es gibt verschiedene Wege, diesen Anforderungen gerecht zu werden. Die Erfindung befasst sich mit dem bekannten Weg, das Kanülenrohr mittels Stromdurchgang so weit, beispielsweise etwa bis auf Rotglut bzw. 500 bis 600 °C (nicht verifizierter Annahmewert), zu erhitzen, dass der Kunststoff des Ansatzes mit dem Kanülenrohr verschmilzt. Hierbei kann man in gewisser Analogie zu einer Schweissverbindung ohne eine zusätzliche Klebkomponente auskommen. Die Erfindung umfasst dabei aber auch eine mechanische Vorverbindung von Kunststoffrohr und Ansatz, die zur Vorfixierung des Kanülenrohres im Ansatz vor dem Verschweissen verwendet werden kann.

Die Erfindung legt speziell einen Stand der Technik nach der US-A-3 437 788 gemäss dem Oberbegriff von Anspruch 1 zugrunde, ergänzt durch Erfahrungen am entsprechenden internen Stand der Technik.

Bei diesem bekannten Verfahren wird zunächst das Kanülenrohr in die übliche Aufnahmebohrung am Ansatz etwa bis in die Stellung eingeschoben, in der das Kanülenrohr mit dem Ansatz verbunden werden soll. An der Innenseite des Ansatzes bildet dieser dabei einen dem später anzubringenden Spritzenhauptkörper zugewandten Becher, in welchen das innere Ende des Kanülenrohres nur geringfügig hineinragt. Unter den Fachleuten ist dabei umstritten, ob ein sehr geringes Hineinragen oder ein etwas grösseres Hineinragen zweckmässig ist. Keinesfalls wird eine sehr grosse freie Wegstrecke innerhalb des Ansatzes erwünscht, um zu vermeiden, dass sich beim Füllen der Spritze zwischen dem inneren Ende des Kanülenrohres und der Ansatzwand ein Lufteinschluss bildet, der beispielsweise Emboliegefahren bei späterem Einspritzen des Spritzeninhalts in einen menschlichen Körper hervorrufen kann. Der Raum innerhalb des Ansatzes vor dem inneren Ende des Kanülenrohres ist daher sehr beengt. Um dort für die Widerstandsaufheizung des Kanülenrohres eine Gegenelektrode anzulegen, hat man die Gegenelektrode stumpf einfach auf das freie innere Ende des Kanülenrohres aufgesetzt.

Es ist auch schon bekannt (DE-A-1 704 102), das innere Ende des Kanülenrohres innerhalb der zentralen Bohrung des Ansatzes enden zu lassen und an der Gegenelektrode einen zentralen Fortsatz auszubilden, der in die zentrale Bohrung des Ansatzes eingreift und mit einer gerundeten Endfläche auf dem Inneren des Kanülenrohres aufsitzt.

An dem mit der Einstichspitze versehenen freien Ende des Kanülenrohres ausserhalb des Ansatzes greift anderseits ein Spannbacken an, der zunächst dazu verwendet werden kann, das Kanülenrohr in den Ansatz einzuschieben und ferner dann in der Endlage das Kanülenrohr gegen die Gegenelektrode anlegt. Der Spannbacken dient bei der gattungsgemässen Konstruktion zugleich als die zweite Elektrode, die zusammen mit der Gegenelektrode dazu dient, eine von einem Schweissspannungsgenerator aufgebrachte Schweissspannung an das Kanülenrohr zum Zwecke dessen kurzfristiger Widerstandserhitzung anzulegen. Je nach Aufheizungstechnik arbeitet man dabei mit einem einmaligen oder mit einem mehrmaligen Stromdurchgangsstoss.

Während des Stromdurchgangs kommt es zur Ausbildung einer mehr oder minder ausgeprägten Funkenerosion zwischen Gegenelektrode und innerem Ende des Kanülenrohres. Typische Erosionslängen sind 0,1 bis 0,3 mm. Die Erosion kann jedoch unter ungünstigen Umständen auch wesentlich erheblicher sein, insbesondere dann, wenn man im gleichen Arbeitsgang Schweissspannung aus demselben Schweissspannungsgenerator an eine Mehrheit oder Vielzahl von Kanülenrohren anlegt und dabei die Verhältnisse bei den einzelnen Kanülenrohren nicht ganz gleichartig eingestellt sind. Bei dem gattungsgemässen Stand der Technik wird daher während des eigentlichen Einschmelzvorganges bzw. des Stromdurchgangs durch das Kanülenrohr der Spannbacken um eine voreingestellte Hubstrecke in Richtung auf die feststellende Gegenelektrode bewegt. Die Hubstrecke kann dabei grössenordnungsmässig 1 mm oder mehr, beispielsweise 3 mm, betragen. Eine Anpassung an die tatsächliche Erosion am inneren Ende des Kanülenrohres, welches ja zwischen Spannbacken und Gegenelektrode eingespannt ist, erfolgt dabei dadurch, dass die Einspannung zwischen Spannbacken und Kanülenrohr reibschlüssig gleitbar gewählt wird und somit während des Schweissvorgangs das Kanülenrohr relativ zum Spannbacken mehr oder minder stark durchrutscht.

Ferner hat der Spannbacken auf der dem Ansatz zugewandten Seite eine trichterförmige Einziehung, welche dazu dient, vereinzelt zugeführte Kanülenrohre zunächst im Spannbacken bequem einführen zu können, bis das jeweilige Kanülenrohr im Spannbacken seine lagerechte Stellung einnimmt und der Spannbacken geschlossen werden kann. Dies bedingt eine Strecke von einigen Millimetern zwischen der Austrittsstelle des Kanülenrohres aus dem Ansatz bis zu der Stelle, an welcher der elektrische Kontakt zwischen Spannbacken und Kanülenrohr hergestellt wird. Diese gattungsgemässe Anordnung führt dazu, dass der Ort des Stromübergangs zwischen Spannbacken und Kanülenrohr nicht genau definiert ist, sich während des Schweissvorgangs ändert und schliesslich relativ erheblichen Abstand zwischen dem Eintrittsort des Kanülenrohres in den Ansatz und dem Ort des tatsächlichen Stromübergangs zwischen Spannbacken und Kanülenrohr ausserhalb des Ansatzes hat. Dies kann zu Ungleichmässigkeiten der Einschmelzbedingungen infolge nicht ausreichend definierter Widerstandsstrecke und bei manchen Metallen des Kanülenrohres zusätzlich zu optisch unerwünschten Anlassfarben des Kanülenrohres an seinem freien Ende ausserhalb des Ansatzes infolge nicht für den Schmelzvorgang wirksam gewordener Glühung des Kanülenrohres führen. Schliesslich kann es auch zu unerwünschten Metallversprödungen im Kanülenrohr kommen.

Der Erfindung liegt die Aufgabe zugrunde, die Einschmelzbedingungen und damit auch die Qualität der hergestellten Einmalkanülen zu verbessern und dabei insbesondere auch eine Anpassung an die Betriebsbedingungen bei Mehrfachherstellung vorzunehmen.

Diese Aufgabe wird bei dem erfindungsgemässen Verfahren gemäss dem Oberbegriff von Anspruch 1 durch das Kennzeichen von Anspruch 1 gelöst. Indem am freien Ende des Kanülenrohres die Schweissspannung nicht mehr im Spannbacken, sondern an einem Ort zwischen dem Ansatz und dem Spannbacken angelegt wird, kann man die Auslegung und Kinematik der mechanisch wirksamen Teile des Spannbackens von den angestrebten Verhältnissen des Stromübergangs am freien Ende des Kanülenrohres entkoppeln und damit auch die elektrischen Übergangsverhältnisse optimieren. Zum Beispiel braucht der Spannbacken nicht mehr als Elektrode elektrisch leitfähig aufgebaut zu sein, sondern kann allein auf seine Einspannfunktion und gegebenenfalls Bewegungsfunktion ausgelegt werden. Beispielsweise kann man weiterhin am Spannbacken einen Einführtrichter für einzeln zugeführte Kanülenrohre auf der dem Ansatz zugewandten Seite vorsehen, ohne dass dabei die wirksame Widerstandslänge des Kanülenrohres ausserhalb des Ansatzes verlängert wird. Vielmehr kann man nunmehr die Stromeinspeisung in das freie Ende des Kanülenrohres unmittelbar im Bereich oder nahe seiner Austrittsstelle aus dem Ansatz vorsehen. Dies vermeidet unnötige Glühvorgänge des freien Endes des Kanülenrohres ausserhalb des Ansatzes und etwa damit verbundene Anlassfarben, die bei Verwendung des bekannten Verfahrens immerhin Längen von 3 bis 5 mm erreichten, wenn man nicht ein Spezialmetall nahm, welches auch bei Glühung nicht anläuft. Die Kanüle kann in diesem Bereich nicht mehr spröde werden oder erweichen, was eine zusätzliche Sicherheit gegen ein Abknicken oder gar Abbrechen des freien Endes des Kanülenrohres, eventuell gar ein Einschiessen einer abgebrochenen Kanülenrohrspitze in eine Vene, darstellt. Schliesslich lässt sich der Übergangswiderstand zwischen der am freien Ende des Kanülenrohres angreifenden Elektrode und dem Kanülenrohr selbst kleinhalten und vergleichmässigen, wobei ohne Rücksicht auf mechanische Einspannbedingungen optimale Kontaktmaterialpaarungen gewählt werden können.

Die Erfindung bietet bereits dann Vorteile, wenn man davon absieht, während des Schweissvorgangs das Kanülenrohr mittels des Spannbackens im Ansatz in Richtung auf die Gegenelektrode zu bewegen, sondern statt dessen die Gegenelektrode zum Ausgleich des Erosionsverlustes zwischen Gegenelektrode und innerem Ende des Kanülenrohres nachstellt, z.B. mittels Federkraft oder einer anderen Nachstellkraft dem erodierenden inneren Ende des Kanülenrohres nachführt. Dann wird man im allgemeinen den Spannbacken und die Anlegstelle der Schweissspannung am freien Ende des Kanülenrohres stationär wählen, behält aber beispielsweise die Vorteile, den Stromübergang und die mechanische Halterung gesondert auslegen zu können, was ihre Geometrie, Materialauswahl sowie die angelegten mechanischen Kräfte betrifft.

Erst recht gelten diese Vorteile, wenn man zwischen dem Spannbacken und/oder der am freien Ende des Kanülenrohres angelegten Elektrode eine Relativverschiebung zulässt, sei es, dass wie bei dem gattungsgemässen Stand der Technik ein vorgegebener Arbeitshub des Spannbackens und damit auch eine Hubbewegung des Kanülenrohres auf die im allgemeinen dann stationäre Gegenelektrode zu vorgesehen wird, sei es, dass man sowohl Gegenelektrode als auch Spannbacken aufeinander abgestimmt nachgiebig lagert.

In diesem Falle kann der Stromübergang mit weitaus geringeren Kräften und im Grenzfall mit stationärer Anordnung des Stromübergangs vorgenommen werden. Beispielsweise kann man für den Stromübergang Kontaktbürsten einsetzen, die für eine mechanische Halterung des Kanülenrohres vollständig ungeeignet wären.

Nun ist es tatsächlich meist bevorzugt, die Gegenelektrode stationär anzuordnen, um sicherzustellen, dass das innere Ende des Kanülenrohres stets einen genau vorbestimmten Überstand innerhalb des becherförmigen Hohlraumes des Ansatzes oder des entsprechenden inneren Bereiches des Ansatzes bei anderer Bauform hat. Wenn dann wie bei dem gattungsgemässen Stand der Technik in der geschilderten Weise beim Anlegen der Widerstandsheizspannung der Spannbacken in Richtung auf den Ansatz verschoben wird, wird vorzugsweise das Anlegen der Widerstandsheizspannung an das freie Ende des Kanülenrohres in konstant bleibendem Abstand vom Ansatz vorgenommen. Trotz der Nachstellbewegung des Kanülenrohres zwecks Kompensation der Funkenerosion am inneren Ende des Kanülenrohres wird dabei während der ganzen Schweisszeit eine konstante Widerstandslänge zwischen der am freien Ende des Kanülenrohres angelegten Elektrode und der Gegenelektrode im Kanülenrohr festgelegt. Dadurch werden die Einschmelzbedingungen erheblich gegenüber dem vorher verwirklichten Verfahren vergleichmässigt. Das gilt insbesondere auch dann, wenn in einem Arbeitsgang mittels derselben Schweissspannung mehrere oder gar eine grössere Vielzahl, z.B. zehn, Kanülen zugleich in ihrem jeweiligen Ansatz eingeschmolzen werden.

Bei dem gattungsgemässen früheren Verfahren wurden die einzeln zugeführten Kanülen zur Aufnahme im Spannbacken zunächst gegen eine Prallfläche geworfen. Dabei prallten die Kanülen gelegentlich mehr oder minder ausgeprägt von der Prallplatte zurück, ehe sie endgültig im Spannbacken erfasst wurden. Um dies auszugleichen, musste man den Hub des Spannbackens in Richtung auf die Gegenelektrode zwecks Erosionsausgleich relativ lang wählen. Die Erfindung wird weiter gefördert, wenn das Kanülenrohr im Spannbacken von einer Hilfskraft in seiner Ausgangslage festgelegt wird, ehe der beim Einschmelzen erforderliche Arbeitshub des Spannbackens erfolgt. Bei der erwähnten Verwendung einer Prallfläche wird dabei vorzugsweise das Kanülenrohr von einer Rückführkraft an der Prallfläche in Anlage gehalten, bis der Spannbacken greift.

Alternativ kann man aber auch statt von einer geschossartigen Zuführung des Kanülenrohres in den Spannbacken eine gesteuerte Zuführung derart verwenden, dass das Kanülenrohr von einer Mitführungskraft an eine das Kanülenrohr im geöffneten Spannbacken ausrichtende Gegenfläche angelegt wird oder in einer anderen definierten Endlage bis zum Ergreifen durch den Spannbacken gehalten wird. Bei Kanülen aus permanent magnetischem oder wenigstens magnetisierbarem Material, die in der Praxis meist nicht verwendet werden, könnte eine derartige Mitführungskraft von einem beispielsweise permanentmagnetischen Führungsmagneten aufgebracht werden. Im Falle magnetisierbaren Kanülenmaterials könnte man auch elektrodynamische Führungskräfte von aussen her einwirken lassen. Man könnte auch daran denken, im Kanülenrohr Wirbelströme zu erzeugen und diesen Zustand von aussen her elektromagnetisch zu führen. Auch ist an eine mechanische oder fluidmechanische Verbindung zwischen Führungskraft und Kanülenrohr bei der Zuführung zu denken. Es wurde bereits erwähnt, dass das erfindungsgemässe Verfahren von erhöhter Bedeutung dann ist, wenn mehrere oder gar eine Vielzahl von Einmalkanülen hergestellt und dabei von einer gemeinsamen Widerstandsheizspannung beaufschlagt werden. Eine derartige gleichzeitige Vielfachherstellung ist an sich im gattungsgemässen Stand der Technik bereits verwirklicht.

Grosse Schwankungen des insgesamt wirksamen Widerstandes für die Widerstandsaufheizung der Kanülenrohre ergeben sich jedoch dann, wenn mindestens ein Kanülenrohr nicht in seine Betriebsstellung für die Einschmelzung gelangt. Dann ist im allgemeinen die Schweissspannung für die übrigen Kanülenrohre überdosiert, und es kommt zu einer Überheizung mit nachteiligen Folgen für die Qualität des Erzeugnisses.

Dem kann in weiterer Verfeinerung der Erfindung gemäss dem Kennzeichen von Anspruch 6 entgegengewirkt werden. Die Verfeinerung der Erfindung ist dabei darin zu sehen, dass die durch die Erfindung angestrebte, mindestens relative Konstanz des für die Widerstandsheizung wirksamen Heizwiderstandes gegenstandslos wird, wenn aus anderer Quelle grosse Störungen der Betriebsverhältnisse und insbesondere der wirksamen Heizleistung auftreten. Die Massnahmen gemäss Anspruch 6 lassen sich jedoch auch vorteilhaft schon bei dem Verfahren gemäss dem gattungsgemässen Stand der Technik einsetzen. Entsprechendes gilt für Anspruch 7, der eine Massnahme angibt, mittels derer einem Ausfall eines Kanülenrohres an seinem Montageort positiv entgegengewirkt wird.

So ist es bei dem gattungsgemässen Stand der Technik bereits vorgesehen, die Kanülenrohre zu magazinieren und von einem Vereinzelungsschieber in Richtung zum jeweiligen Spannbacken zu entnehmen. Dabei kann es jedoch dazu kommen, dass das Kanülenrohr am Vereinzelungsschieber verklemmt oder aus anderen Gründen aus diesem nicht frei kommt. Um einer derartigen Blockade des Nachschubs von Kanülenrohren entgegenzuwirken, wird vorzugsweise eine am im Vereinzelungsschieber liegenden Kanülenrohr reibschlüssig einwirkende Entnahmekraft aufgebracht, welche das Kanülenrohr zwangsweise auswerfen kann.

Grundsätzlich lässt sich eine Verschweissung des thermoplastischen Materials des Ansatzes am Kanülenrohr schon bei hinreichend engem losen Einstecken des Kanülenrohres in der zentralen Bohrung des Ansatzes erreichen. Man kann aber gemäss Anspruch 8 die Güte der Verbindung verbessern und die Herstellungstaktzeiten verkleinern, wenn man vor dem Verschweissungsvorgang das Kanülenrohr in enge Anlage am Ansatz aufweitet. Der Eingriff kann dabei reibschlüssig oder vorzugsweise formschlüssig sein, was an sich zur ausschliesslich mechanischen Verbindung von Kanülenrohr und Ansatz bekannt ist (FR-A-1 357 757). Wenn es auch in Sonderfällen ausreichen mag, wenn die mechanische Verbindung das Kanülenrohr im Ansatz nur zugfest oder nur druckfest fixiert, so wird doch eine Festigkeit sowohl gegen Zug- als auch gegen Druckkräfte gemäss Anspruch 9 bevorzugt. Anspruch 10 gibt einen bevorzugten Festigkeitsbereich an. Auch eine gute Abdichtung lässt sich bei entsprechender Aufweitung in alle Radialrichtungen erhalten. Die erforderliche Zugfestigkeit hängt vom Kanülendurchmesser ab. Bei dünnen Kanülen (z.B. 0,5 bis 0,8 mm Aussendurchmesser) wird eine Zugfestigkeit von 8 kp reichen. Bei dickeren Kanülen erreicht man Zugfestigkeiten zwischen 10 und 20 kp oder mehr.

Das Kanülenrohr kann in eine vorgeformte Aussparung im Ansatz aufgeweitet werden. Dann braucht man Material des Ansatzes gar nicht oder nur in einem relativ geringen Umfang zu verdrängen. Man kann jedoch auch ausschliesslich unter Materialverdrängung arbeiten.

In vielen Fällen wird verdrängtes Material nach Aufweitung des Kanülenrohres zu einer Verformung der seitlichen Aussenkontur des Ansatzes führen. Dieser kann man gemäss Anspruch 12 entgegenwirken. Man braucht also nur bei der vorgefertigten Gestalt des Ansatzes dort Material auszusparen, wo bei der Aufweitung des Kanülenrohres Material verdrängt wird.

Für eine allein reibschlüssige Vorfixierung kann die zentrale Bohrung im Grenzfall über die ganze Länge der Eingriffsstrecke des Kanülenrohres im Ansatz konstanten Bohrungsquerschnitt haben. Vorzugsweise wird jedoch gemäss Anspruch 13 vor dem inneren Ende des Kanülenrohres eine Einschnürung der zentralen Bohrung vorgesehen. Diese Einschnürung kann im Vormontagezustand zum losen Auflagern des Kanülenrohres dienen und nach dem Aufweiten des Kanülenrohres zur Abstützung des inneren Endes des Kanülenrohres mit herangezogen werden und so die Druckfestigkeit des Kanülenrohres im Ansatz sicherstellen. Wenn vor der Aufweitung das Kanülenrohr einen etwas kleineren lichten Querschnitt als die Einschnürung hat, kann man dabei die Bemessung so vornehmen, dass der Dorn gerade durch die lichte Öffnung der Einschnürung der zentralen Bohrung im Ansatz passt und das Innenrohr gerade so weit aufweitet, dass die lichte Weite des Kanülenrohres gleich der lichten Weite der Einschnürung wird.

Man kann aber auch die Aufweitung noch um ein gewisses Mass weitertreiben und dabei davon ausgehen, dass der zunächst mit aufgeweitete Anteil der Einschnürung nach dem Aufweitvorgang am Kanülenrohr sich wieder um ein mehr oder minder grosses Mass elastisch rückstellt.

Bei dieser Anordnung mit Einschnürung, aber auch allgemein, ist es vorteilhaft, wenn das innere Ende des Kanülenrohres nicht aus dem Basisteil des Ansatzes, welcher das Kanülenrohr umschliesst, nach innen zur Spritze hin um eine gewisse Strecke hervorsteht. Es kann dadurch sicher vermieden werden, dass an der Einmündungsstelle des Kanülenrohres in den Spritzenraum im Anschlussbereich des Ansatzes an den Spritzenzylinder ein Totraum zwischen einem hervorstehenden Ende des Kanülenrohres und der Wandfläche des Ansatzes verbleibt. Es ist zwar an sich bekannt, die Gegenelektrode noch innerhalb einer zentralen Bohrung des Ansatzes stumpf auf das innere Ende eines Kanülenrohres aufzusetzen (DE-A-1 704 102); die Erfindung sieht darüber hinaus noch die Möglichkeit vor, die Gegenelektrode an der Innenfläche des Kanülenrohres anzulegen und so unabhängig von der Lage des inneren Endes des Kanülenrohres den Kontaktort genau einstellen zu können. Man kann gegebenenfalls sogar gemäss Anspruch 16 den zum Aufweiten des Kanülenrohres verwendeten Dorn auch als Gegenelektrode verwenden.

Die Erfindung bezieht sich auch auf eine Vorrichtung zum Herstellen von Einmalkanülen aus metallischen Kanülenrohren und Ansätzen aus Kunststoff gemäss dem Oberbegriff von Anspruch 18, wie sie einleitend bereits zur Erläuterung des gattungsgemässen Verfahrens beschrieben wurde. Die erfindungsgemässe Vorrichtung lässt sich zur Ausführung des erfindungsgemässen Verfahrens nutzen und gibt bevorzugte konstruktive Mittel zur Ausführung der einzelnen Verfahrensschritte an.

Um zunächst die Widerstandsheizspannung an das freie Ende des Kanülenrohres zwischen dem Ansatz und dem Spannbacken anlegen zu können, ist im Kennzeichen von Anspruch 18 eine an bestimmtem Ort wirksam werdende Elektrodenzange vorgesehen. Diese bietet Vorteile gegenüber der bereits erwähnten möglichen Kontaktbürstenanordnung, indem sie sich bedarfsweise leicht schliessen und lösen lässt, mit den mechanischen Schliesselementen bedarfsweise selbst als Elektrode dienen kann oder alternativ hinreichend stabil ausbildbar ist, um in ihrem Kontaktflächenbereich eine gesonderte Stromzuführung zu besitzen, und ferner gewährleistet, Schliessdruck mindestens zweiseitig und bedarfsweise auch die Spannungsanlegung mindestens zweiseitig durchführen zu können. Vorzugsweise hat dabei mindestens ein Backen der Elektrodenzange, vorzugsweise haben dies beide Backen, eine etwa punkt- oder linienförmige Kontaktfläche. Die Kontaktfläche kann dabei von einem an das Material des Kanülenrohres kontaktpaarungsmässig angepassten Kontaktmaterial gebildet sein, mit dem der jeweilige Schliessbacken der Elektrodenzange, vorzugsweise auswechselbar, belegt ist.

Wenn der Spannbacken auf den Anordnungsort des Ansatzes in seiner Halteeinrichtung zu mit Reibschluss gegenüber dem Kanülenrohr verschiebbar ist, um Erosion des inneren Kanülenendes bei angelegter Schweissspannung auszugleichen, dann wird vorzugsweise die Elektrodenzange eng benachbart der Austrittsstelle des Kanülenrohres aus dem Kunststoffansatz stationär und mit leichterem Reibschluss als der Spannbacken gegenüber dem Kanülenrohr angeordnet. Um Verkantungen zu vermeiden, wird allgemein die Elektrodenzange frei schwimmend gelagert. Dabei gibt es weder eine unzweckmässige Wechselwirkung mit Vorschubbewegungen des Stammbackens noch Störungen bei Schliess- und Öffnungsbewegung der Elektrodenzange. Diese ist zweckmässig in ihrer Schliessstellung federvorgespannt und mit einer Öffnungsnockensteuerung versehen.

Wenn ferner zur Zuführung vereinzelter Kanülenrohre eine Gleitstrecke für diese, beispielsweise eine mittels der Schwerkraft wirksame Gleitschiene oder ein pneumatischer Zuführungsschlauch, an einer Prallfläche hinter dem geöffneten Spannbacken mündet, wird zur Vermeidung eines eine unbestimmte Endlage verursachenden Hochprallens des auftreffenden Kanülenrohres auf der Prallwand zweckmässig eine das jeweilige Kanülenrohr auf die Prallfläche zu mitnehmende Friktionswalze vorgesehen, die zweckmässigerweise zwischen dem Spannbacken in seiner Aufnahmestellung und der Prallfläche ortsfest oder, alternativ, im Spannbacken gelagert wird.

Anspruch 24 beschreibt eine bevorzugte Vorrichtung zur Erfassung der Anzahl der jeweils für die Einschmelzung bereiten Kanülenrohre und zur entsprechenden Einstellung der jeweils erforderlichen Schweissleistung. Dabei wird davon ausgegangen, dass selbst bei Abwesenheit eines Ansatzes allenfalls durch unnötiges Glühen des betreffenden Kanülenrohres Ausschuss produziert wird, der später aussortiert werden kann, während beim Leerglühen eines solchen Kanülenrohres immerhin noch die Betriebsbedingungen der übrigen Kanülenrohr-Ansatz-Paare unverändert gut bleiben.

Anspruch 25 sieht eine bevorzugte Ausführungsform der bei Anspruch 24 vorgesehenen Erfassungseinrichtung der vorhandenen Kanülenrohre unter Verwendung paralleler Widerstandsmessstrecken durch Teilabschnitte der Kanülenrohre vor. Alternativ könnte man auch andere Detektoren verwenden, wobei nur als eines unter der Vielzahl der dem Fachmann zur Verfügung stehenden Detektoren photoelektrische Detektoren erwähnt seien.

Um schliesslich positiv sicherzustellen, dass bei Verwendung eines Kanülenrohrmagazins mit Vereinzelungsschieber ein sicheres Auswerfen der Kanülenrohre aus dem Vereinzelungsschieber und damit eine möglichst ununterbrochene regelmässige Zufuhr von Kanülenrohren erfolgt, sieht Anspruch 26 eine bevorzugte konstruktive Lösung zum Aufbringen einer reibschlüssigen Entnahmekraft auf im Vereinzelungsschieber liegende Kanülenrohre vor. Hierzu wird eine im Vereinzelungsschieber gelagerte Friktionswalze verwendet. Diese ist vorzugsweise nach Anspruch 27 so gelagert und ausgebildet, dass sie die Aufnahme des jeweiligen Kanülenrohres im Vereinzelungsschieber nicht behindert, jedoch zusätzlich zu ihrer reibschlüssigen Schleuderwirkung noch in der Abgabestellung als Ausheber für das Kanülenrohr aus dem Vereinzelungsschieber dient.

Die nach dem erfindungsgemässen Verfahren oder der Vorrichtung nach der Erfindung hergestellten Einmalkanülen zeigen auch in der Serie eine gleichmässige ausserordentliche Festigkeit zwischen Kanülenrohr und Ansatz, lassen sich so herstellen, dass einwandfreie Dichtheit gewährleistet ist, zeigen eine wesentliche Verbesserung hinsichtlich Materialerweichung oder -versprödung, ermöglichen eine lagekonstante Anordnung des Kanülenrohres im Ansatz, lassen sich bei einer Vielzahl von Materialpaarungen von Ansatz und Kanülenrohr einsetzen und sind auch optisch ansehnlich wegen des Fortfalls oder einer mindestens praktischen Reduzierung von Anlassfarben am freien Ende des Kanülenrohres. Für die Kontaktgabe lassen sich angepasste wertvolle Materialien gezielt und damit kostensparend verwenden, und trotz des scheinbaren Mehraufwandes lassen sich gegebenenfalls sogar Einsparungen durch auf die Einspann- und Bewegungsfunktion abgestellte Auslegungen des Spannbackens erreichen. Es lassen sich dabei auch solche Kunststoffe für den Ansatz verarbeiten, die nicht zur Wasseraufnahme neigen.

Die Erfindung lässt sich ferner bei einer Vielzahl von Materialien und geometrischen Formen der Ansätze sowie Materialien und Formen der Kanülenrohre verwenden, wie sie von verschiedenen Herstellern ganz unterschiedlich angeboten werden. Bevorzugt verwendet werden Ansätze aus Polyamid 6 oder Polyamid 66 und/oder Kanülenrohre aus V2A- oder V4A-Stahl.

Die Spannbacken lassen sich bei konstantem Gleithub im Verhältnis zum Kanülenrohr aus gut gleitfähigem, abriebfestem Material, mindestens im Spannbereich, herstellen, so dass auch die sonst auftretenden Gleitriefen reduziert werden können. Für das Kontaktmaterial der Zange lässt sich entweder Messing, z.B. bei Herstellung der ganzen Zange aus Messing, oder auch Silber, hier vorzugsweise als Zangenbelag, verwenden. Überhaupt kann man für die Kontaktfläche der mit dem freien Ende des Kanülenrohres zusammenwirkenden Elektrode gut geeignetes Paarungsmaterial, beispielsweise Silber oder auch Wolfram, verwenden. Wolframstifte werden, wie schon beim gattungsgemässen internen Stand der Technik, zweckmässig als Gegenelektroden verwendet. Dabei kann man die Gegenelektrode entweder stumpf auf das innere Ende des Kanülenrohres aufsetzen oder auch einen für den elektrischen Kontaktübergang geeigneten geometrischen Verlauf, z.B. in Gestalt eines trichterförmig an der Gegenelektrode eingezogenen Konus, verwenden.

Die Erfindung wird im folgenden anhand schematischer Zeichnungen an einem Ausführungsbeispiel noch näher erläutert. Es zeigen:

Fig. 1 einen vertikalen Schnitt durch wesentliche Teile einer erfindungsgemässen Vorrichtung;

Fig. 2 eine Draufsicht auf die Elektrodenzange gemäss Fig. 1;

Fig. 3 einen ausschnittweisen und teilweise geschnittenen, teilweise schematisierten Längsschnitt durch eine Magaziniereinrichtung für Kanülenrohre gemäss Fig. 1;

Fig. 4 ein Schaltbild einer in Verbindung mit der Vorrichtung gemäss den Fig. 1 bis 3 verwendbaren elektrischen Einrichtung zum Einstellen der Einschmelzleistung in Abhängigkeit von der Anzahl der an verschiedenen Betriebsstellen einer Mehrfacheinrichtung für die Einschmelzung bereitgestellten Kanülenrohre;

Fig. 5 einen axialen Querschnitt durch Kanülenrohr und Ansatz im unverbundenen Zustand mit einem Aufweitdorn;

Fig. 6 einen axialen Halbschnitt einer Alternative zu Fig. 5 mit einem Halter für den Ansatz;

Fig. 7 eine aus der Anordnung von Fig. 6 gewinnbare aufgeweitete Form des Kanülenrohres nebst Ansatz im axialen Halbschnitt;

Fig. 8a und 8b – zu Fig. 7 alternative aufgeweitete Formen im Längsquerschnitt einer Wand des jeweiligen Kanülenrohres;

Fig. 9a und 9b – zwei alternative Formen des Ansatzes, dessen äusseres Ende unterschiedlich vorgeformt ist, jeweils unter zusätzlicher Veranschaulichung eines zylindrisch aufgeweiteten Endes des Kanülenrohres;

Fig. 10 bis 15 – Längsschnitte durch den Ansatz und das Kanülenrohr bei einer ersten Art des Aufweitens des Kanülenrohres mit nachfolgendem Hinformen von Kunststoffmaterial des Ansatzes vor das innere Ende des Kanülenrohres in verschiedenen Arbeitsphasen, und die

Fig. 16 bis 18 – eine Alternative hierzu, wiederum in verschiedenen Arbeitsphasen.

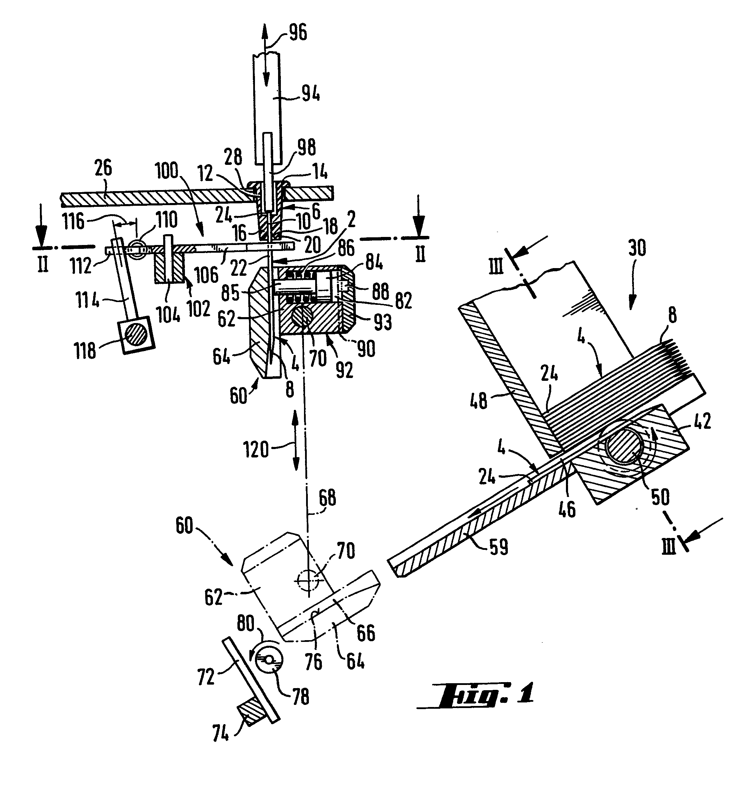

In Fig. 1 ist zunächst eine mögliche unter einer Vielzahl in Frage kommender Bauformen einer Einmalkanüle 2 in der Stellung zu sehen, in der ihr Kanülenrohr 4 aus Metall in den Ansatz 6 aus thermoplastischem Kunststoff eingeschmolzen werden kann. Das Kanülenrohr 4 ist ein dünnes Röhrchen mit einer Einstechspitze 8 am äussersten freien Ende. Das Kanülenrohr 4 tritt durch eine zentrale Bohrung 10 des Ansatzes 6 in dessen von einem becherförmigen Teil 12 des Ansatzes begrenzten Innenraum hinein. Am freien Ende des Ansatzes befindet sich eine flanschförmige Erweiterung 14 zur Verbindung mit dem eigentlichen Spritzenkörper. Die zentrale Bohrung 10 des Ansatzes 6 befindet sich in dessen Basis 16, von der der becherförmige Teil 12 ausgeht. Die Basis 16 ist an der Seite, an der das freie Ende des Kanülenrohres 4 aus dem Ansatz 6 austritt, bei 18 etwas eingezogen, so dass der dem freien Ende 22 des Kanülenrohres 4 zugewandte Ringkragen 20 die Austrittsstelle des freien Endes 22 aus dem Ansatz 6 beschreibt. Das innere Ende 24 tritt innerhalb des vom becherförmigen Teil 12 des Ansatzes 6 umgebenen Innenraumes des Ansatzes 6 um eine kleine vorgegebene Strecke aus der Basis 16 heraus. Das freie Ende 22 des Kanülenrohres 4 hat hier eine grössere Länge als der Ansatz 6. Ausserdem ist die Länge des vom becher- oder topfförmigen Teil 12 des Ansatzes 6 umschlossenen Innenraumes des Ansatzes 6 we-.

sentlich grösser als die Länge des inneren Endes des Kanülenrohres. Dieser Innenraum des Ansatzes 6 kann zentralsymmetrisch in bezug auf das Kanülenrohr gestaltet sein.

Neben der beschriebenen Bauform einer Einmalkanüle 2 gibt es eine Vielzahl anderer Bauformen, auf welche die Erfindung analog anwendbar ist.

Die zentrale Bohrung 10 des Ansatzes 6 ist dabei so bemessen, dass sie das Kanülenrohr 4 so eng umfasst, dass bei Aufheizung des Kanülenrohres 4 das umgebende Wandmaterial der Basis 16 auf das Kanülenrohr aufgeschmolzen wird.

Die Einschmelzvorrichtung weist zunächst eine Trägerplatte 26 mit einer Aufnahmeöffnung 28 für eine Mehrzahl, z.B. zwei oder vorzugsweise mehr, z.B. fünf oder zehn, Ansätze 6 auf, welche in die Aufnahmeöffnung 28 unter Abstützung ihrer flanschförmigen Erweiterung 14 auf der Trägerplatte 26 eingehängt werden können.

Die anderen Aufnahmestellen sind in Richtung normal zur Zeichnungsebene hinter der gezeichneten Aufnahmestelle 28 fortgesetzt zu denken. Wenn auch in Fig. 1 nur eine Vorrichtung zum Einschmelzen eines einzigen Kanülenrohres 4 in seinem Ansatz 6 dargestellt ist, so ist auch bezüglich der anderen Elemente von Fig. 1 eine entsprechende Vervielfachung normal zur Zeichnungsebene hinzuzudenken.

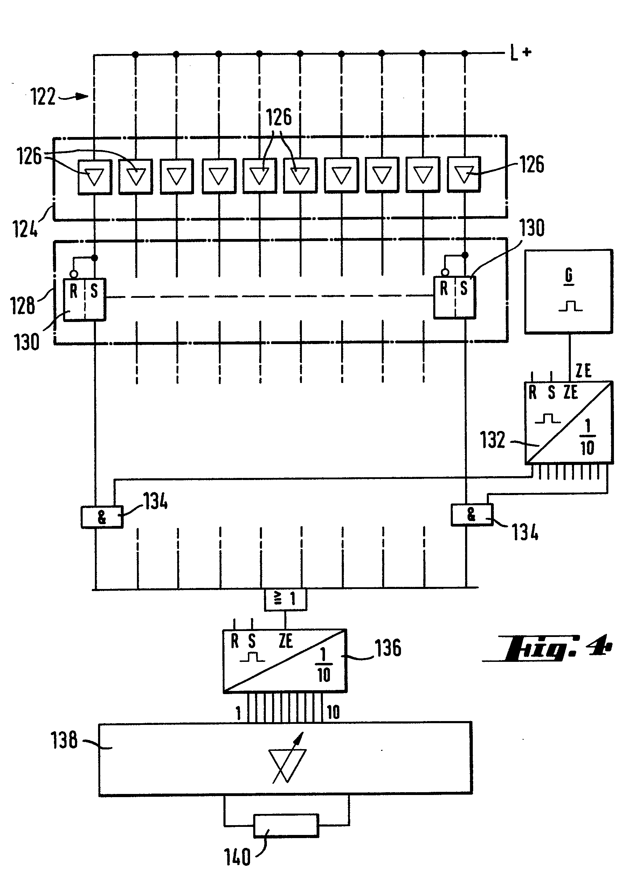

Die Kanülenrohre 4 werden zunächst in einem Kanülenrohrmagazin 30 (vgl. auch Fig. 3) bereitgestellt. Jeder Arbeitsstelle der Vielfachanordnung ist dabei ein gesonderter Speicherraum 32 für Kanülenrohre 4 zugeordnet. Jeder Speicherraum 32 weist dabei ein eigentliches grösseres Speichervolumen 34 auf, welches über eine mindestens einseitige Schräge 36 in einen unteren Zuführraum 38 eingeengt ist, an dessen Boden Aufnahmekammern 40 für einzelne Kanülenrohre eines längs des Doppelpfeiles 44 um das in Fig. 3 dargestellte Mass hin und her verschiebbaren Vereinzelungsschiebers 42 ausgebildet sind. Der Hin- und Herhub des Vereinzelungsschiebers ist so bemessen, dass jeweils ein Kanülenrohr 4 in der Aufnahmekammer 40 des Vereinzelungsschiebers bis unter den Eingang 46 einer weiterführenden Gleitrinne transportiert werden kann. Die Anordnung ist dabei so getroffen, dass die einzelnen Kanülenrohre 4 in der Gleitrinne 46 mit ihrem inneren Ende vorab weitertransportiert werden. Dies ist in Fig. 1 besonders deutlich zu sehen, wo die Einsteckspitzen 8 der einzelnen Kanülenrohre 4 der als Eingang dienenden Gleitrinne 46 abgewandt dargestellt sind. Man erkennt dabei, dass das jeweilige Kanülenmagazin 30 etwa so schräg eingestellt ist, dass sich die inneren Enden 24 der Kanülenrohre 4 zunächst an einer geradlinigen Stützwand 48 abstützen und ausrichten.

In dem Vereinzelungsschieber 42 ist eine erste Friktionswalze 50 um ihre Achse drehbar gelagert und mindestens während des Entnahmevorganges eines Kanülenrohres aus dem Kanülenrohrmagazin 30 in Umdrehung versetzbar (siehe eingezeichneter Pfeil).

Die Friktionswalze 50 hat ferner je einen Abschnitt 52 konstanten grösseren Durchmessers und einen axial längeren Abschnitt 54 konstanten kleineren Durchmessers.

Die Abschnitte 52 und 54 wechseln miteinander ab und sind symmetrisch zueinander durch konische Übergangsstücke 56 bzw. 58 fallenden bzw. steigenden Durchmessers miteinander verbunden.

Darüber hinaus ist die erste Friktionswalze 50 längs der Hubstrecke des Vereinzelungsschiebers zusätzlich noch axial verschiebbar.

Die Anordnung ist dabei so getroffen, dass bei der Aufnahme eines Kanülenrohres 4 in der zugehörigen Aufnahmekammer 40 des Vereinzelungsschiebers 42 der Grund der Aufnahmekammer 40 vom Abschnitt 54 kleineren Durchmessers der Friktionswalze gebildet ist. In dieser Stellung lässt sich das Kanülenrohr 4 vollständig im Vereinzelungsschieber aufnehmen und mit diesem zum Eingang 46 der weiterführenden Gleitschiene 59 weitertransportieren. Der Übergang aus der Aufnahmekammer 40 in den Eingang 46 wird dabei nicht nur dadurch gefördert, dass die Friktionswalze in Umlauf ist oder in Umlauf gesetzt wird, sondern auch noch durch eine zusätzliche Aushubbewegung des Kanülenrohres 4 aus der Aufnahmekammer 40 in den Eingang 46 infolge zusätzlicher axialer Verschiebung der ersten Friktionswalze 50 relativ zum Vereinzelungsschieber. Dies hat die Wirkung, dass zunächst je nach axialer Verschieberichtung der eine oder der andere konische Übergangsabschnitt 56 oder 58 die Tiefe der Aufnahmekammer 40 einengt, bis schliesslich im – nicht immer erforderlichen – Grenzfall der Abschnitt grossen Durchmessers 52 die Aufnahmetiefe der Aufnahmekammer 40 wesentlich oder ganz reduziert hat. Beim Rückhub des Vereinzelungsschiebers erfolgt dann der umgekehrte Vorgang in die beschriebene Ausgangslage.

Die an den Eingang 46 anschliessende Gleitschiene 59 ist gemäss Fig. 1 schräg nach unten geneigt, so dass die einzelnen Kanülenrohre 4 unter Beibehaltung ihrer magazinierten Lage allein unter der Schwerkraft weitergefördert werden. An die Stelle der Gleitschiene 59 kann auch ein geneigter Rohr- oder Schlauchförderer oder gegebenenfalls auch ein Fluidförderer, insbesondere pneumatischer Förderer, treten. Beispielsweise kann man den Gleitkanal rohrförmig schliessen und den Eingang 46 mit Druckluft beaufschlagen.

Fluchtend mit der Gleitschiene 59 ist ein Spannbacken 60 so angeordnet, dass das auf der Gleitschiene 59 ankommende Kanülenrohr 4 in einen Aufnahmekanal 66 zwischen zwei Backenteilen 62 und 64 eintritt. Der untere Backenteil 64 ist dabei gemäss Fig. 1 in Richtung zur Gleitschiene 59 verlängert und am Grund des Aufnahmekanals 66 etwas nach aussen zu angeschrägt oder gekrümmt, um einen guten Eintritt des ankommenden Kanülenrohres in den Aufnahmekanal zu gewährleisten. Eine entsprechende trichterartige Krümmung kann auch an dem kürzeren Backenteil 62 vorgesehen sein.

Der Spannbacken 60 ist fluchtend mit der Gleitschiene 59 in strichpunktierten Linien gezeichnet und befindet sich hierbei in einer unteren geneigten Stellung. Er kann in die obere voll ausgezeichnete und geschnitten dargestellte Stellung in Fig. 1 angehoben und gleichzeitig geschwenkt werden. Die Hubstrecke ist durch die strichpunktierte Linie 68 angedeutet. Die Schwenkung erfolgt über Schwenkzapfen oder eine Schwenkwelle 70.

In der unteren strichpunktierten Stellung ist der Aufnahmekanal 66 des Spannbackens 60 zunächst offen, so dass das herabgleitende Kanülenrohr mit seinem inneren Ende frei durch den Aufnahmekanal 66 hindurchfallen und gegen die Prallfläche einer stationär angeordneten Prallplatte 72 fallen kann, die von einem Haltebalken 74 getragen ist. Zwischen der Prallplatte 72 und der Gleitbahn 76 im unteren Backenteil 64, also dem Grund des Aufnahmekanals 66, ist im Wege des ankommenden Kanülenrohres 4 eine zweite Friktionswalze 78 drehbar stationär gelagert und in Richtung des Pfeiles 80 so angetrieben, dass sie ein etwa von der Prallfläche zurückprallendes Kanülenrohr wieder in Anlage an die Prallfläche bringt und an dieser in Anlage hält.

Im oberen Backenteil 62 ist ein pneumatischer Zylinder 82 mit einem in ihm geführten Kolben 84 ausgespart, der einen schmaleren Kolbenstössel 85 trägt, dessen freies Ende zusammen mit dem oberen Backenteil 64 einen Spannbacken zum Festspannen des an der Prallfläche ausgerichteten Kanülenrohres 4 innerhalb des Aufnahmekanals 66 bildet.

Der Kolben ist durch eine Kolbenfeder 86 normalerweise so nach aussen vorgespannt, dass der Aufnahmekanal 66 frei bleibt. Seine in der oberen ausgezogenen Stellung in Fig. 1 dargestellte Schliessstellung erfolgt pneumatisch unter Beaufschlagung durch Druckluft über einen Zuführungskanal 88 zur äusseren Stirnfläche des Kolbens 84.

Bei Vielfachanordnung ist es möglich, und wie in der oberen Darstellung in Fig. 1 durch eine zusätzliche Strichpunktlinie grob schematisch angedeutet, zwischen dem Körper 92 des oberen Backenteils und einem gemeinsamen Zylinderdeckel 93, in welchem der Zuführungskanal 88 ausgespart ist, eine gemeinsame Betätigungsmembran 90 für die einzelnen nebeneinander in einzelnen Zylindern angeordneten Kolben 84 einzuspannen.

Für die verschiedenen Kolben-Zylinder-Einheiten 82, 84 kann dann je ein einziger gemeinsamer oberer Backenteil 62 und unterer Backenteil 64 vorgesehen sein. Die Begriffe «oben» und «unten» beziehen sich dabei auf die strichpunktierte untere Stellung des Spannbackens 60 nach Fig. 1.

Sobald das in jedem zugeordneten Aufnahmekanal 66 angekommene Kanülenrohr an der gemeinsamen Prallplatte 72 ausgerichtet ist, kann der Stössel 85 gegen das am Backenteil 64 widergelagerte Kanülenrohr 4 gedrückt werden, so dass dieses im Spannbacken transportfest eingespannt ist. Der Spannbacken wird dann in die obere ausgezogen dargestellte Stellung in Fig. 1 überführt. Dabei wird das Kanülenrohr 4 fluchtend auf die zentrale Bohrung 10 im Ansatz 6 ausgerichtet und ausserdem in einem Hubvorgang mit seinem inneren Ende 24 in die in Fig. 1 oben dargestellte Endstellung im Ansatz 6 eingeschoben.

Oberhalb der Aufnahmeöffnung 28 in der Trägerplatte 26 ist ein stabförmiger Stromleiter 94 entsprechend dem Doppelpfeil 96 auf und ab verschiebbar. Der Stromleiter 94 trägt eine Gegenelektrode 98 aus Wolfram, die unter weitgehender Ausfüllung des schmalen Innenraums des Ansatzes 6 innerhalb von dessen becherförmigem Teil 12 auf das innere Ende 24 des Kanülenrohres stumpf oder mit anderer geeigneter übergangswiderstandsarmer und definierter Position aufsetzt. Das Kanülenrohr 4 wird dabei zwischen der Gegenelektrode 98 und dem Spannbacken 60 eingespannt.

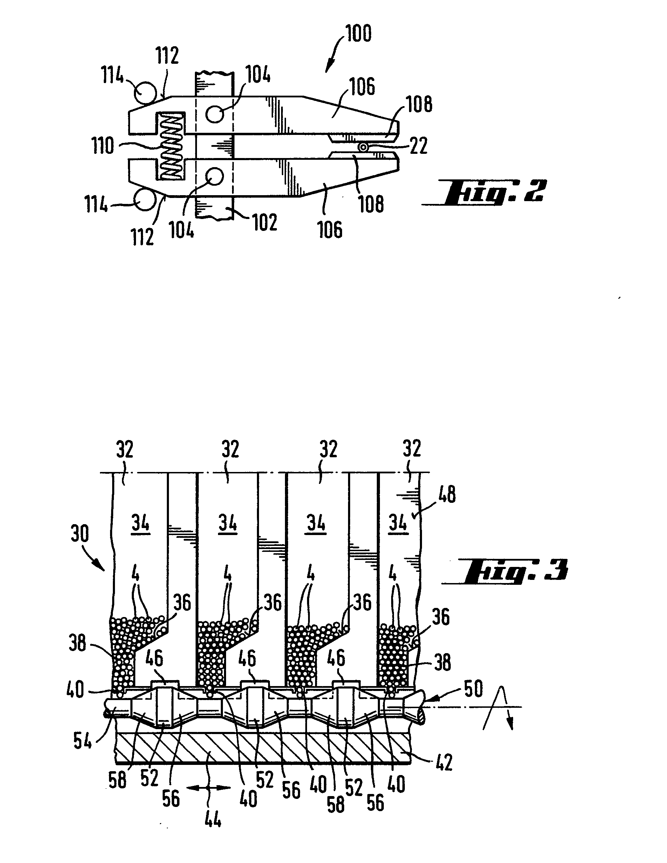

Eine in Fig. 2 mehr im einzelnen dargestellte Elektrodenzange 100 bildet die mit der Gegenelektrode 98 zusammenwirkende andere Elektrode, mittels derer Schweissspannung an den zwischen den beiden Elektroden liegenden Abschnitt des Kanülenrohres 4 angelegt und dieses so erhitzt werden kann, dass der angrenzende Kunststoff im Ansatz auf das Kanülenrohr aufgeschmolzen wird. Beide Elektroden können hierzu an einen Schweissspannungsgenerator angeschlossen werden, der mit relativ niederen Spannungswerten im Bereich einiger Volt, jedoch relativ hohen Stromwerten in der Grössenordnung etlicher Ampère arbeitet.

Zweckmässig wird eine nicht gezeigte Spannungsstabilisierungseinrichtung vorgesehen, um die Betriebsbedingungen an jeder Schweissstelle, auch bei gleichzeitiger Schweissung an mehreren Schweissstellen, zu gewährleisten. Wenn die spezifischen Widerstände und geometrischen Bedingungen sehr schwanken, kann man alternativ oder zusätzlich auch in der jeweiligen Widerstandsschweissstrecke den Schweissstrom auf einen Sollwert einregeln.

Die Elektrodenzange ist in einem schwimmend allseitig frei nachgiebigen Lager 102 auf zwei Lagerstiften 104 so stationär gehalten, dass die Elektrodenzange 100 horizontal ausgerichtet ist und mit ihren beiden Zangenhälften 106 um die beiden Lagerstifte 104 schwenkbar ist. Im Zangenkopf liegen sich zwei vorzugsweise austauschbare Kontaktbeläge 108 gegenüber, zwischen denen das freie Ende 24 des Kanülenrohres 4 einspannbar ist. Es kann dabei eine beliebig geeignete Kontaktfiguration gewählt werden, und zwar ausser der dargestellten flachen Kontaktgabe, welche zwei linienförmige Kontaktflächen ergibt, vorzugsweise auch mit zwei punktförmigen Kontaktflächen zur genauen Festlegung der Kontaktstelle. Man kann allerdings auch in jedem Kontaktbelag eine an den Umfang des Kanülenrohres etwas angepasste ausgenommene Gestaltung vorsehen.

Anstelle relativ weicher Kontaktbeläge 108, z.B. aus Ag, kann man auch harte Beläge, z.B. aus W, vorsehen, gegebenenfalls gar auf gesonderte Kontaktbeläge verzichten und die ganze Zange, mindestens ihren Backenbereich, aus vorzugsweise hartem Kontaktmaterial herstellen.

Der Kontaktdruck wird durch eine den Zangenkopf schliessende leichte Druckfeder 110 aufgebracht, deren Kontaktdruck sehr klein im Verhältnis zur Einspannkraft zwischen dem Backenteil 64 und dem Kolbenstössel 85 gewählt werden kann.

Zum Öffnen der Elektrodenzange 100 weist diese an den beiden hinteren Enden der Zangenhälften 106 je eine Kurve bzw. Keilfläche 112 auf, gegen die jeweils ein in Öffnungsrichtung der Zange verschiebbarer Nockenzapfen 114 einwirken kann. Die zum Öffnen der Zange erforderliche Schwenkbewegung des Nockenzapfens 114 ist durch den Doppelpfeil 116 in Fig. 1 kenntlich gemacht. Sie erfolgt durch beschränkte Verschwenkung des Nockenzapfens 114 durch eine im Takt hin und her bewegte Betätigungswelle 118.

Manchmal wird es als nachteilig empfunden, wenn die Öffnungs- und/oder Schliessbewegungen der Zange zu schlagartig erfolgen. Dann ist es empfehlenswert, Öffnung und/oder Schliessung sanft über eine Steuerkurve, z.B. Kulissenführung, Schlitzführung oder Nockenbahn, zu steuern.

Der Spannbacken 60 führt zweimal einen vertikalen Arbeitshub durch. In der ersten Phase wird er zum Einführen des Kanülenrohres in die in Fig. 1 dargestellte Stellung im Ansatz längs der Hubstrecke 68 nach oben bewegt und dabei, wie geschildert, in die Einführstellung geschwenkt. Die zweite Phase erfolgt während des Schweissstromdurchganges durch den an den Schweissstromgenerator angeschlossenen Kontaktbelag 108, dann durch den durch den Ansatz ragenden Bereich des Kanülenrohres sowie die ebenfalls an den Schweissspannungsgenerator angeschlossene Gegenelektrode 98. Diese zweite Hubphase ist durch den Doppelpfeil 120 übertrieben kenntlich gemacht. Die Einspannfläche des Kolbenstössels 85 sowie die Einspannfläche am Backenteil 64 gleiten dabei unter Überwindung der Einspannkraft längs des an der stationären Gegenelektrode 98 abgestützten Kanülenrohres um eine unbestimmte Strecke, da während des Stromdurchgangs zugleich eine gewisse Erosion zwischen Gegenelektrode und innerem Ende 24 des Kanülenrohres 4 erfolgt, welche jedoch durch die zweite Phase der Aufwärtsbewegung des Spannbackens ausgeglichen wird. Da der Spannbacken keine stromübertragenden Aufgaben hat, kann man die am Kanülenrohr angreifenden Einspann- und Gleitflächen entsprechend optimal auslegen, z.B. aus einem geeigneten Gleitmaterial, welches wenig Riefen hervorruft, gegebenenfalls sogar ausser aus in Frage kommenden Metallen auch aus Kunststoff, z.B. Polytetrafluoräthylen.

Da auch die Elektrodenzange stationär angebracht ist, muss auch das freie Ende 22 des Kanülenrohres 4 relativ zu den Kontaktflächen des Kontaktbelages 108 gleiten können. Die Gleitstrecke ist dabei durch die Erosion zwischen innerem Ende 24 des Kanülenrohres und Gegenelektrode bestimmt. Es entsteht dabei infolge der schwachen Federkraft der Druckfeder 110 nur ein geringer Gleitwiderstand, der wesentlich kleiner gehalten werden kann als die Einspannkraft des Kanülenrohres im Spannbacken 60. Anderseits befindet sich während des ganzen Stromdurchgangs zwischen Gegenelektrode 98 und Kontaktbelag 108 stets eine genau vorbestimmte konstante Länge des Kanülenrohres zwischen den beiden Elektroden, so dass ihr wirksamer Heizwiderstand des Einschmelzvorganges unabhängig von der Länge des Erosionsabbrandes am inneren Ende 24 des Kanülenrohres konstant bleibt.

Da die Elektrodenzange am Kanülenrohr unmittelbar unter dessen Austritt aus dem Ansatz 6 zwischen diesem und der höchsten Stelle des Hubs des Spannbackens angreift, ist ausserdem die Widerstandsstrecke ausserhalb des Ansatzes minimal gehalten, so dass das Kanülenrohr praktisch überhaupt nur noch im Bereich der Einziehung 18 oder etwas unter dieser farblich anlaufen kann. Die Einziehung kann man jedoch so wählen, dass sie beim Einschmelzvorgang durch Kunststoff geschlossen wird und es praktisch überhaupt nicht mehr zu einem ungenutzten Glühvorgang in solchen Bereichen kommt, die zwischen der Einstechspitze 8 des Kanülenrohres 4 und dem Ansatz 6 liegen. Da gerade dieser Bereich bei Benutzung der Spritze mechanisch stark beansprucht ist, kann man ihn viel mehr als früher von Versprödungen oder Erweichungen freihalten und auch durch Glühen sonst entstehende Anlauffarben vermeiden.

Wenn das Kanülenrohr eingeschmolzen ist, kann der Spannbacken 60 wieder geöffnet und in seine strichpunktierte untere Ausgangsstellung gemäss Fig. 1 wieder zurückgeführt werden, um den Zyklus zu wiederholen. Entsprechend wird der Stromleiter 94 so weit zurückgezogen, dass die Gegenelektrode 98 aus dem Ansatz heraustritt. Dann kann die fertige Einmalkanüle 2 mittels der Trägerplatte 26, die beispielsweise an einem grossen Drehteller angeordnet ist, zu einer weiterverarbeitenden Station abgeführt werden.

An der abgebrochenen Darstellung von Fig. 3 ist bereits auf eine Vervielfachung der Stationen gemäss Fig. 1 und 2 durch mehrfache Einzeichnung von Speicherräumen 32 des Kanülenrohrmagazins 30 und Eingängen 46 Bezug genommen, die jeweils zu einer Gleitschiene 59 gemäss Fig. 1 führen. Bei einer derartigen vielfachen, z. B. zehnfachen, Anordnung sind dementsprechend natürlich auch zehn Elektrodenzangen 100, Zylinder 82, Aufnahmeöffnungen 28 usw. vorgesehen.

Um zu erfassen, ob jeweils ein Kanülenrohr in seine Betriebsstellung gelangt, kann man eine elektrische Widerstandsmessung zwischen dem als eine Messelektrode ausgebildeten Spannbacken 60 und einzelnen gegeneinander isolierten Abschnitten der Prallplatte 72 vorsehen, wobei dann zweckmässig die Friktionswalze 78 aus elektrisch nichtleitendem Material gebildet wird. Alternativ könnte man auch die Friktionswalze 78 als Gegenmesselektrode wählen. Man kann dann abfragen, auf welchen Messstrecken ein elektrischer Widerstand vorhanden ist, und die für alle Einschmelzungen erforderliche gesamte Schweissleistung entsprechend einstellen.

Eine entsprechende elektrische Schaltung des einerseits mit dem stromführenden Teil der Elektrodenzange 100 und anderseits mit der Gegenelektrode 98 verbundenen Schweissspannungsgenerators zeigt Fig. 4.

In Fig. 4 sind zunächst als Beispiel einer Vielfachanordnung zehn Kontrollstellen 122 dargestellt; allgemein kann man eine unbestimmte Anzahl n derartiger Kontrollstellen vorsehen entsprechend der Anzahl gleichzeitig einzuschmelzender Kanülenrohre in Ansätzen.

Die Kontrollstellen befinden sich zwischen dem Spannbacken 60 und den einzelnen gegeneinander isolierten Kontaktstellen an der Prallfläche 72.

Bei Anlegung einer Spannung zwischen dem beispielsweisen positiven Pol L+ und einem Gegenpol an der Verstärkereinrichtung 124 erfolgt durch alle die Kanülenrohre ein Stromdurchgang, welche sich in ihrer eingespannten Stellung befinden. Die beim Stromdurchgang erfolgenden Signale werden in einem jeder Widerstandsmessstrecke zugeordneten Verstärker 126 so verstärkt, dass sie in einem Kontrollspeicher 128 jeweils ein RS-Flip-Flop setzen können. Dort, wo kein Stromdurchgang erfolgt, wird das Flip-Flop nicht aus seiner Grundstellung gesetzt. Man kann also mit einem einzigen Messvorgang zunächst das tatsächliche Bild, wo Kanülenrohre vorhanden sind, im Kontrollspeicher 128 in den einzelnen Flip-Flops 130 abbilden.

Es ist ferner ein Taktgenerator G vorgesehen. Dessen Taktimpulse stellen einen Abfragezähler 132 schrittweise weiter, so dass an seinen zehn Ausgängen nacheinander Abfrageimpulse abgegeben werden. Diese werden jeweils mit Hilfe einzelner UND-Schaltungen 134 mit den Ausgangssignalen des zugehörigen Flip-Flops 130 des Kontrollspeichers 128 verknüpft und in einem Kontrollzähler 136 registriert. In Abhängigkeit vom registrierten Zählergebnis wird ein Leistungssteller 138 so eingestellt, dass am Schweissgerät 140 jeweils eine an die Zahl der in der Betriebsstellung befindlichen Kanülenrohre angepasste Schweissleistung bereitgestellt wird. Dadurch kann man erreichen, dass für jede Kanüle, unabhängig davon, ob an den anderen Betriebsplätzen noch Kanülen sind, jeweils eine vorbestimmte Schweissleistung bereitgestellt wird.

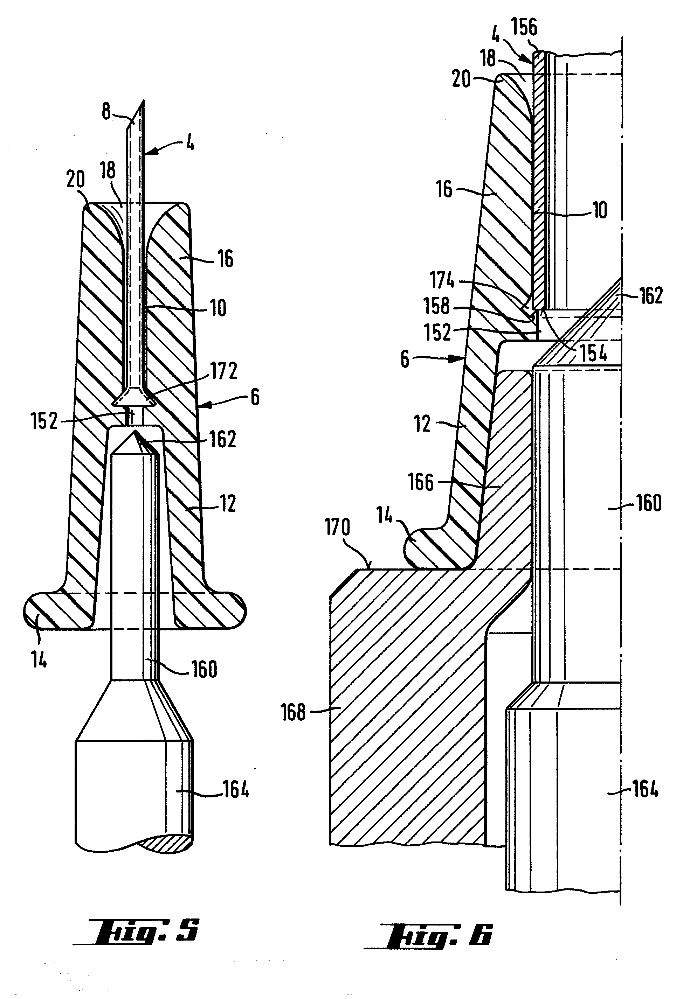

Der für die Widerstandsschweissung erforderliche enge Kontakt zwischen Kanülenrohr 4 und Ansatz 6 kann zweckmässig durch Vorfixierung so hergestellt werden, wie es an verschiedenen Modifikationen anhand der Fig. 5 bis 9 dargestellt ist.

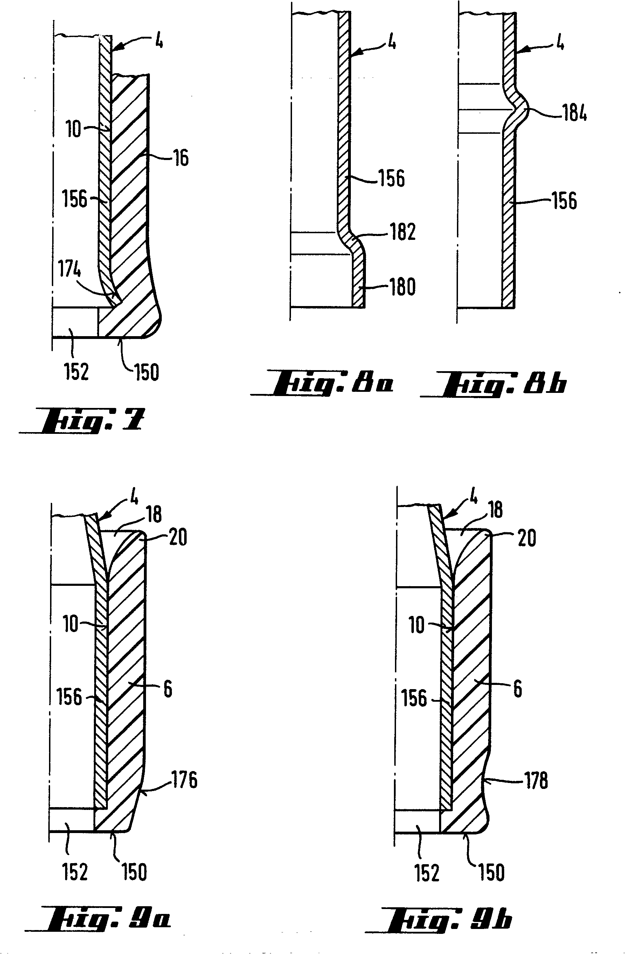

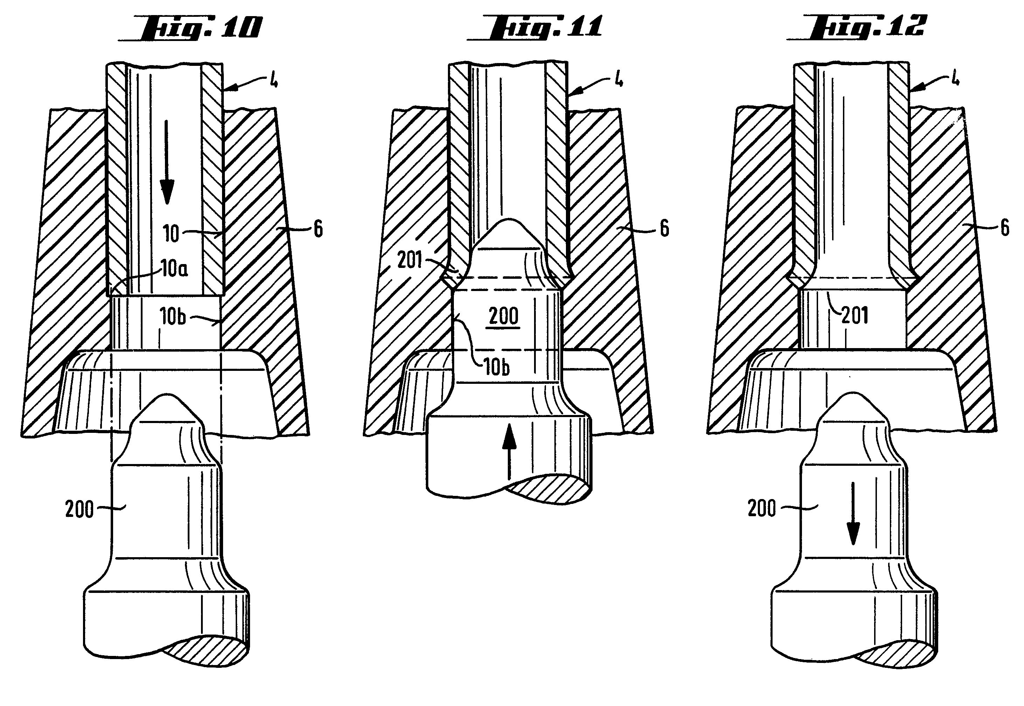

In den Fig. 5 und 6 hat dabei der Ansatz 6 dieselbe Aussenform wie in Fig. 1. Entsprechendes gilt für Fig. 7 und Fig. 9a und 9b, wo aber der becher- oder topfförmige Teil 12 fortgelassen ist und die Basis 16 mit der stirnseitigen Anschlussfläche 150 direkt an den Zylinder einer Spritze, z.B. durch Schweissen, angesetzt werden kann. Die Besonderheiten der Fig. 5 bis 9b liegen in der inneren Gestaltung der zentralen Bohrung in der Basis 16, der dort gewählten Form des Kanülenrohres 4 sowie in möglichen Aussenformen des Ansatzes vor der endgültigen Verbindung mit dem Kanülenrohr gemäss den Fig. 9a und 9b.

Die Besonderheiten der Fig. 5 und 6 lassen sich sinngemäss auch auf die Fig. 7 und 9a und 9b und umgekehrt, übertragen. Ebenso sind die Alternativen der Fig. 8a und 8b sowohl bei der Form des Ansatzes 6 gemäss Fig. 5 und 6 als auch bei den möglichen Ansatzformen der Fig. 7 und 9a und 9b einsetzbar.

Bei allen Fig. 5 bis 9 wird davon ausgegangen, dass das Kanülenrohr 4 zunächst lose in die zentrale Bohrung 10 eingesteckt wird und zur Herstellung einer engen Umfassung durch die Basis 16 des Ansatzes 6 anschliessend aufgeweitet wird.

Abweichend von der Anordnung gemäss Fig. 1 ragt dabei das Kanülenrohr 4 nicht bis in den freien Raum des becher- oder topfförmigen Teils 12 hinein, sondern endet bereits vor einer Einschnürung 152 der zentralen Bohrung 10 an deren Übergang in den becher- oder topfförmigen Teil 12 gemäss Fig. 5 oder 6 bzw. am Anschlussende 150 an eine Spritze gemäss Fig. 7 oder 9. Am vorteilhaftesten ist es, wenn gemäss Fig. 6 die lichte Weite der Kanüle 4 relativ zur lichten Weite der Einschnürung 152 und der sonstigen Bohrung 10 im Basisteil 16 so gewählt wird, dass gemäss Fig. 6 die Stirnfläche 154 der Wand 156 des Kanülenrohres 4 nur mit ihrer Aussenend-Ringfläche auf einem ringförmigen Absatz 158 der Einschnürung 152 in der Bohrung 10 aufsitzt, während die innenliegende Ringzone der Wand 156 des Kanülenrohres der lichten Öffnung der Einschnürung 152 gegenüberliegt. Dies erfordert sehr enge Toleranzen der zusammenwirkenden Teile, da übliche Wandstärken eines Kanülenrohres bei etwa 1,0 oder 1,5 mm liegen, bei Kanülenaussendurchmessern zwischen 0,6 mm (und weniger) und 2,0 mm (und mehr). 0,6 und 2,0 mm geben Unter- und Obergrenze der meistens verwendeten Kanülen an, wobei die tatsächlichen Masse eher noch im mittleren Bereich der angegebenen Grenzzahlen liegen.

Alternativ kann man aber auch, wie es eher in Fig. 5 angedeutet ist, von vornherein die lichte Weite des Kanülenrohres nicht kleiner als die lichte Weite der Einschnürung 152 wählen.

Im Gegensatz zu Fig. 1, also gemäss den Ausführungsformen der Fig. 5 bis 9, endet hier jeweils das innere Ende des Kanülenrohres 4 noch innerhalb des Basisteils 16 und ragt nicht, wie in Fig. 1, in einen freien Raum an der Rückseite des Basisteiles hinaus, wie es in Fig. 1 zur Erleichterung des stumpfen Aufsetzens der Gegenelektrode 98 vorgesehen ist.

Das an der Einschnürung 152 zunächst aufgelagerte Kanülenrohr 4 wird dann im Ansatz durch Aufweiten mechanisch vorfixiert. Zum Aufweiten dient ein Aufweitdorn 160, der zweckmässig mit einer konischen Spitze 162 versehen ist. Der wirksame Teil des Aufweitdornes 160 wird zweckmässig so bemessen, dass er entweder gerade durch die lichte Weite der Einschnürung 152 passt (Fig. 6), oder etwas gar erheblich im Durchmesser weiter (Fig. 5), als die lichte Weite der Einschnürung 152 bemessen ist. Im Fall einer Überweite im Verhältnis zur lichten Weite der Einschnürung 152 wird beim Aufweitvorgang davon ausgegangen, dass das Kunststoffmaterial der Einschnürung so nachgiebig ist, dass der Aufweitdorn 160 nicht nur zum Aufweiten des Kanülenrohres 4 dient, sondern auch zunächst das Kunststoffmaterial der Einschnürung 152 im Basisteil 16 des Ansatzes 6 verdrängen kann. Man kann dann eine mehr oder minder grosse Elastizität zur Rückstellung des Kunststoffmaterials in den Ausgangszustand nach beendigter Aufweitung des Kanülenrohres vorsehen.

Der Aufweitdorn 160 ist an einem ausserhalb des Ansatzes 6 auch während des Aufweithubes verbleibenden Schaft 164 befestigt bzw. mit diesem aus einem Stück bestehend. Der Schaft 164 ist mit einer nicht näher dargestellten konventionellen Hubbetätigung verbunden. Der Aufweitdorn 160 ist so bemessen, dass er bei Ansätzen 6 mit becher- oder topfförmigem Teil 12 gemäss Fig. 5 oder 6 mit erheblichem Spiel noch in den inneren Freiraum des Teils 12 passt. In diesem Freiraum kann gemäss Fig. 6 ein Haltestutzen 166 eines Halteteils 168 einer Montagemaschine greifen. Der Halteteil 168 kann dabei Bestandteil eines Schalttellers sein. Die Anordnung kann dabei gemäss Fig. 6 so getroffen sein, dass die Aussenfläche des Haltestutzens 166 mit der Innenfläche des Teils 12 im wesentlichen formschlüssig zusammenwirkt und gegebenenfalls zusätzlich, oder alternativ, der becherförmige Teil 12 mit seiner flanschförmigen Erweiterung 14 auf einer zugewandten Stirnfläche 170 des Halteteils 168 aufliegt. Die innere Bohrung des Haltestutzens 166 kann zugleich als Führung für den Aufweitdorn 160 dienen.

Es ist möglich, nur eine Aufweitung eines relativ kurzen Abschnittes am inneren Ende des Kanülenrohres 4 vorzunehmen, indem man für den Aufweitvorgang nur die Spitze 162 des Aufweitdornes 160 benutzt. Dabei entsteht eine trompeten- oder glockenförmige Aufweitung 172 gemäss Fig. 5. Diese Aufweitung kann entweder unter Verdrängung von Wandmaterial der zentralen Bohrung 10 im Basisteil 16 oder in eine vorgeformte, der Aufweitungsform komplementäre Erweiterung 174 der zentralen Bohrung gemäss Fig. 6 erfolgen.

Fig. 6 zeigt dabei das Kanülenrohr 4 im noch nicht aufgeweiteten Zustand, Fig. 7 im aufgeweiteten Zustand bei vorgeformter Erweiterung 174 der Bohrung 10 und Fig. 5 das gegen eine elastische Verformung aufgeweitete Kanülenrohr.

Wenn auch in Fig. 5 bei der elastischen Verformung der Einfachheit halber keine zugehörige Verformung der Aussenkontur des Ansatzes 6 dargestellt ist, so kann doch durch Materialverdrängung eine derartige Verformung der Aussenkontur erfolgen. Zu deren völliger oder teilweiser Kompensation kann man entweder gemäss Fig. 9a eine Anschrägung 176 oder gemäss Fig. 9b eine Taille 178 in der vorgefertigten Form des Ansatzes 6 dort vorsehen, wo beim Aufweiten des Kanülenrohres Material des Ansatzes verdrängt wird. Im Idealfall erhält man dann im aufgeweiteten Zustand die Konfiguration von Fig. 5, bei der man keinerlei Materialverdrängung nach aussen mehr an der Aussenkontur des Ansatzes erkennen kann. Die Gestalt der am vorgefertigten Ansatz vorgesehenen äusseren Aussparung hängt natürlich von der gewählten Verformung des Kanülenrohres und der Lage dieser Verformung im Ansatz sowie von den Materialcharakteristika des Kunststoffes des Ansatzes ab.

Anstelle einer im wesentlichen konischen Aufweitung am inneren Ende des Kanülenrohres 4 kann man dieses im Grenzfall, wie anhand von Fig. 9a und 9b auch dargestellt, auch über seine ganze Eingriffsstrecke der zentralen Bohrung 10 zwischen Einschnürung 152 und Austritt aus dem Ansatz 6 zylindrisch aufweiten. Ein sehr vorteilhafter Kompromiss zwischen zylindrischer Aufweitung und Aufweitung nur am inneren Ende des Kanülenrohres zeigt Fig. 8a, wo das Kanülenrohr nicht nur mit der Spitze 162 des Aufweitdorns 160, sondern auch teilweise von dessen Schaft 164 aufgeweitet ist und dementsprechend an seinem inneren Ende einen zylindrischen Anfangsabschnitt 180 aufweist, der dann über einen konischen Übergangsabschnitt 182 in einen nicht aufgeweiteten Bereich des Kanülenrohres 4 übergeht.

Bei der Anordnung gemäss den Fig. 9a und 9b ist das Kanülenrohr 4 nur durch Auflagerung auf der Schulter der Einschnürung 152 der zentralen Bohrung 10 formschlüssig gegen Druck in Richtung in die Spritze abgestützt, während die Abstützung gegen Zug nur reibschlüssig erfolgt. Bei den anderen Ausführungsformen erfolgt eine formschlüssige Abstützung in beiden Richtungen, da auch die Zugfestigkeit durch Formschluss des Kanülenrohres im Basisteil 16 des Ansatzes 6 sichergestellt ist.

Alternativ zur dargestellten Aufweittechnik kann man auch eine andere, beispielsweise von der Herstellung von Kugelschreiberminen bekannte, Aufweittechnik verwenden, bei der innerhalb des im Basisteil 16 gelegenen Bereichs des Kanülenrohres eine Sicke 184 ausgeformt wird. Bei dieser Anordnung ist sogar die Einschnürung 152 entbehrlich.

Der Aufweitdorn 160 kann bei entsprechender Gestaltung des Schaftes 164 auch die Funktion der Gegenelektrode 98 gemäss Fig. 1 übernehmen.

Für eine Vorfixierung des Kanülenrohres 4 im Ansatz 6 reicht es beispielsweise aus, dass das Kanülenrohr mindestens gegen eine Zugkraft von 6 kp zugfest montiert ist. Vorteilhafter sind noch etwas grössere Zugfestigkeiten. Im Grenzfall kann man Zugfestigkeiten von 6 kp, 8 kp oder weit mehr erreichen.

Voraussetzung dabei ist sorgfältiges Arbeiten. mit genauen Toleranzen. Im allgemeinen reicht es aus, bei der Aufweitung den Aussendurchmesser des Kanülenrohres etwa um $1/_{10}$ mm, in manchen Fällen auch weniger, zu erweitern. Vorzugsweise erfolgt eine Erweiterung um mindestens mehrere zehntel Millimeter, zweckmässig mehr als 20% des Kanülenaussendurchmessers. Das Material des Ansatzes, z.B. auch ein Polycarbonat, muss mit einer solchen Aufweitung verträglich sein, falls es beim Aufweiten verdrängt wird.

Es ist zweckmässig, beim Aufweiten den Aufweitdorn in das Kanülenrohr unter Vibration einzuführen. Man kann den Dorn in Axialrichtung oder in Umfangsrichtung vibrieren. Man kann auch einen rotierenden Dorn axial vibrieren. Zweckmässig sind niederfrequente Vibrationen mit einer Frequenz von 10 Hz an aufwärts.

Bei den Ausführungsformen des Ansatzes 6 gemäss den Fig. 5 bis 9b kann die Einschnürung 152 der Bohrung 10 vom Aufweitdorn 160 beim Aufweiten des Kanülenrohres 4 mehr oder minder stark mit aufgeweitet werden, und es hängt dann von der Rückstellkraft und Güte der Elastizität des verdrängten Materials der Einschnürung 152 ab, inwieweit diese sich nach Herausziehen des Aufweitdornes 160 wieder zurückstellt und ihre Funktion als Abstützung des Kanülenrohres 4 gegen Druck und gegebenenfalls als Abdichtung des Kanülenrohres gegenüber dem Ansatz wieder übernimmt.

Diese Schwierigkeit kann man vermeiden, wenn die Bohrung 10 zunächst ganz ohne Einschnürung 152, z.B. als Bohrung mit über die Länge konstantem Querschnitt, oder mit einer so weit bemessenen Einschnürung 152 gefertigt wird, dass diese vom Aufweitdorn nicht selbst aufgeweitet wird. In derartigen Fällen ist es möglich, die Abstütz- und Dichtfunktion der Einschnürung 152 folgendermassen zu verbessern:

Zunächst wird ein Abschnitt des Kanülenrohres 4, welches bis in die gewünschte Einbaulage in die Bohrung 10 eingesteckt ist, aufgeweitet und so das Kanülenrohr zunächst mindestens gegen Zugbeanspruchung im Ansatz 6 festgelegt. Danach wird Wandmaterial der Bohrung 10 im Ansatz 6 in einen verformbaren, gegebenenfalls fluiden, Zustand erwärmt und vor das innere Ende des Kanülenrohres 4 geschoben, um dort eine das Kanülenrohr gegen Druck abstützende Einschnürung 152 erst zu bilden oder eine schon vorhandene Einschnürung zu verstärken bzw. mit einem kleineren lichten Querschnitt auszubilden. Das verschobene Wandmaterial kann dabei auch zu einer Verklebung des Kanülenrohres 4 im Ansatz 6 verwendet werden.

Es ist meist erforderlich, die Innenfläche des Kanülenrohres von Kunststoffmaterial freizuhalten und ferner einen lichten Strömungskanal in der Bohrung 10 des Ansatzes 6 hinter dem inneren Ende des Kanülenrohres freizulassen. Hierzu kann ein gesonderter Abdichtdorn verwendet werden, der abdichtend in das innere Ende des Kanülenrohres 4 eingeführt werden kann, sobald das Kanülenrohr 4 aufgeweitet ist. Zur Erwärmung des Kunststoffmaterials des Ansatzes 6 im Wandungsbereich der Bohrung 10 hinter dem inneren Ende des Kanülenrohres 4 kann dann ein auf dem Abdichtdorn, vorzugsweise thermisch isoliert, geführter hohler Heizstempel dienen, der nach Abdichtung des inneren Endes des Kanülenrohres 4 und hinreichender Erwärmung der zu verschiebenden Kunststoffmasse diese dann in ihre gewünschte Endlage verschiebt.

In dem beschriebenen Fall ist es zweckmässig, das Aufweiten des Kanülenrohres 4 an einer ersten Arbeitsstation und das Verschieben des Kunststoffes an einer zweiten Arbeitsstation vorzunehmen, um so schnelle Taktzeiten zu erreichen.

Man kann aber auch den Aufweitdorn 160 zugleich als Abdichtdorn verwenden und den Heizstempel auf dem Aufweitdorn 160 führen. In diesem Falle ist es zweckmässig, Aufweitung und Kunststoffverschiebung an derselben Arbeitsstation vorzunehmen.

Der Abdichtdorn bzw. der Aufweitdorn 160 verbleibt so lange in Eingriff mit dem inneren Ende des Kanülenrohres 4, bis sich der verschobene Kunststoff wieder hinreichend verfestigt hat.

Heizstempel und Abdichtdorn oder Aufweitdorn können ein einziges Werkzeug bilden, bei dem der Abdichtdorn oder Aufweitdorn mit Vorlauf gegenüber dem Heizstempel aktiv wird.

Die Fig. 10 bis 15 zeigen eine Vorrichtung, bei der eine Aufweitung und nachfolgende Hinformung von Kunststoffmaterial des Ansatzes vor das Kanülenrohr mit zwei aufeinanderfolgenden Werkzeugen 200 und 204, zweckmässig an aufeinanderfolgenden Arbeitsstationen, vorgenommen wird, während die Fig. 16 bis 18 eine alternative Möglichkeit unter Verwendung eines einzigen Werkzeuges 220, zweckmässig an derselben Arbeitsstation, zeigen.

Bei der ersten Alternative ist im Ansatz 6 eine durchgehende Bohrung 10 vorhanden, die über die ganze Länge konstanten Querschnitt haben kann. In diesem Falle kann die Positionierung des Kanülenrohres 4 in seiner Montagelage im Ansatz durch äussere Haltemittel sichergestellt werden. Man kann aber auch, wie hier gezeigt, zur Festlegung der Endlage einen kleinen Absatz 10a in der Bohrung vorsehen.

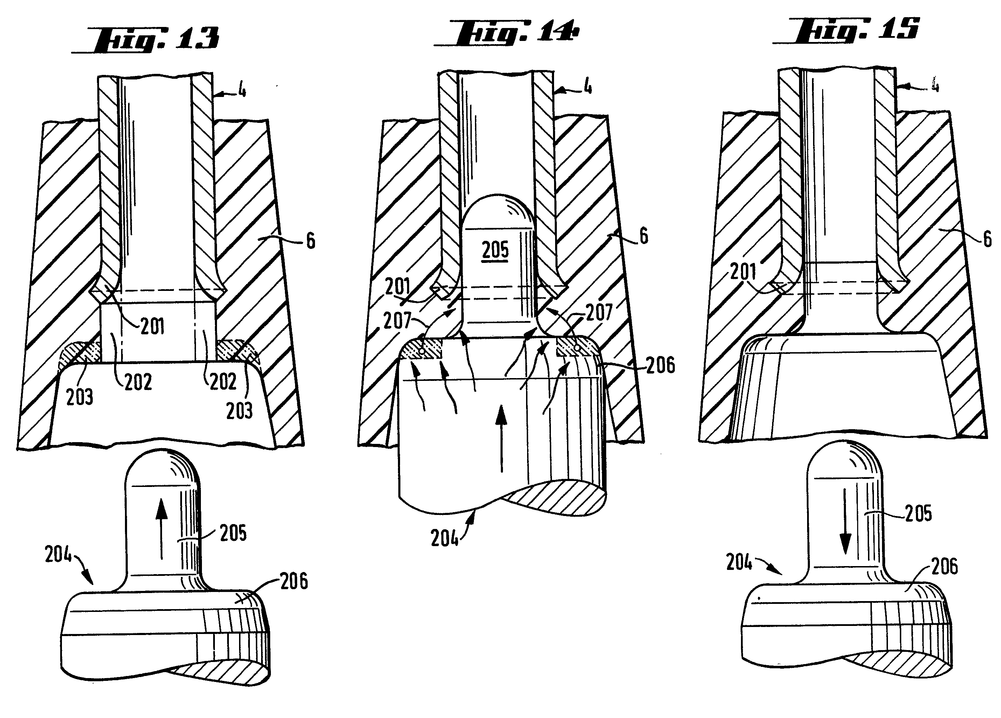

Zum Aufweiten des Kanülenrohres wird ein Aufweitdorn 200, dessen Aussendurchmesser dem Innendurchmesser der Bohrung 10 hinter der Endlage des inneren Endes des Kanülenrohres 4 wenigstens annähernd entspricht (passend in Bohrungsabschnitt 10b), aus der Ausgangslage von Fig. 10 (nach Einschieben des Kanülenrohres 4 gegen den Absatz 10a in Pfeilrichtung), in die Aufweitstellung gemäss Fig. 11 axial in Pfeilrichtung verschoben, bis sich das innere Ende 201 des Kanülenrohres 4 trompetenartig entweder gegen das Material des Ansatzes oder in eine Aussparung in demselben aufgeweitet hat. Dann wird der Aufweitdorn 200 gemäss Fig. 12 wieder in eine Ausgangslage ausser Eingriff zurückgezogen.

In der nächsten Arbeitsstation wird das zweite Werkzeug 204 gemäss Fig. 13 in Position gebracht. Es weist einen vorsprungartigen Dichtansatz 205 und einen ringförmigen Heizansatz 206 auf. Ziel ist es, die Zone 202 durch Kunststoffmaterial aufzufüllen, welches sich in der vorgeformten Form des Ansatzes 6 in der Zone 203 befindet. Hierzu wird der Dichtansatz 205 zunächst gemäss Fig. 14 in das innere Ende des Kanülenrohres 4 abdichtend eingeschoben. Obwohl der Dichtansatz 205 hier mit der heizkolbenartigen Heizeinrichtung integral ausgebildet sein kann, kann man auch den Dichtungsstift 205 axial innerhalb eines gesondert angefertigten Heizkolbens führen. Sobald die Heizeinrichtung die Stellung gemäss Fig. 14 erreicht hat, ist das Kunststoffmaterial aus dem Ansatz in Richtung der Pfeile 207 in die Endstellung verformt. Man erkennt, dass der Dichtungsdorn 205 sogleich auch Kalibrierdorn für die Fortsetzung des Innenkanals des Kanülenrohres ist.

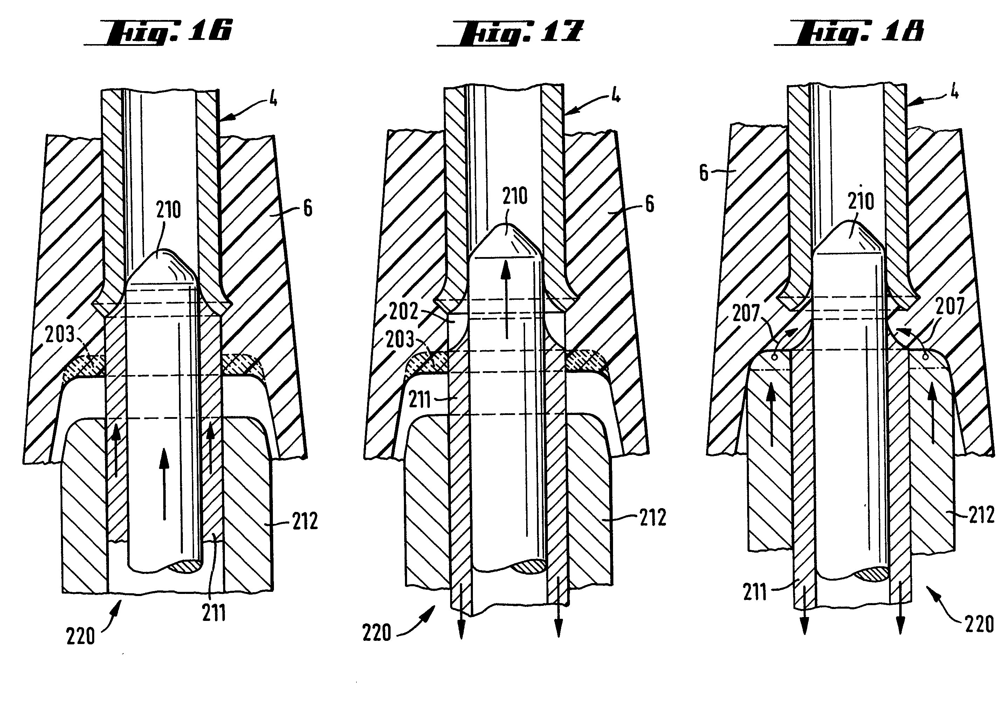

Fig. 15 zeigt die entformte Endstellung.

Bei der alternativen Konstruktion gemäss Fig. 16 bis 18 weist das einzige Werkzeug 220 einen axial innenliegenden Aufweitdorn 210, eine diesen umgebende, gegebenenfalls aus thermisch isolierendem Material bestehende Schiebehülse 211 sowie einen auf dieser wiederum axial geführten hohlen Heizkolben 212 auf.

Gemäss Fig. 16 wird in einer ersten Betriebsphase das Kanülenrohr 4 im vorher beschriebenen Sinne durch die Einheit 210, 211, die zusammen als Aufweitdorn dient, aufgeweitet.

In der nächsten Betriebsphase gemäss Fig. 17 wird der innere Teil des Aufweitdornes 210 in eine abdichtende Position in das Kanülenrohr weiter eingeschoben, während im wesentlichen gleichzeitig die Formhülse 211 axial etwas zurückgezogen wird. Dadurch entsteht zwischen dem aufgeweiteten inneren Ende des Kanülenrohres 4 und dem gegenüberliegenden Ende der Hülse 211 ein Freiraum 202, der gegebenenfalls durch nicht dargestellte Öffnungen mit einem auch nicht dargestellten inneren Entlüftungskanal innerhalb des Stempels 210 in Verbindung treten kann. Man kann auch andere Entlüftungen vorsehen, beispielsweise Längsrinnen in der Führung zwischen Hülse 211 und Dorn 210.

In dritter Phase erfolgt dann ein Fliess-Pressvorgang unter thermischer Mitwirkung des aufgeheizten Heizstempels 212. Dabei wird wiederum entsprechend dem Pfeil 217 Material vor das innere Ende des Kanülenrohres 4 geformt.

Der Aufweitdorn hat vorzugsweise einen zylindrischen Querschnitt und eine kegelförmige oder nach innen geschwungene scharfe Spitze.

**Patentansprüche**

1. Verfahren zum Herstellen von Einmalkanülen (2) unter Einschmelzen eines metallischen Kanülenrohres (4) in einem Ansatz (6) aus Kunststoff durch Einschieben des Kanülenrohres in den Ansatz, Einspannen des Kanülenrohres zwischen einer das innere Ende (24) des Kanülenrohres widerlagernden Gegenelektrode (98) und einem am freien Ende (22) des Kanülenrohres angreifenden Spannbacken (60) sowie zeitweises Anlegen einer Widerstandsheizspannung zwischen der Gegenelektrode und dem aus dem Ansatz herausragenden freien Ende des Kanülenrohres und gleichzeitiges Verschieben von Spannbacken und Gegenelektrode relativ aufeinander zu, dadurch gekennzeichnet, dass die Widerstandsheizspannung an das freie Ende (22) des Kanülenrohres (4) zwischen dem Ansatz (6) und dem Spannbacken (60) angelegt wird.

2. Verfahren nach Anspruch 1, bei dem beim Anlegen der Widerstandsheizspannung der Spannbacken (60) in Richtung auf den Ansatz (6) verschoben wird, dadurch gekennzeichnet, dass das Anlegen der Widerstandsheizspannung an das freie Ende (22) des Kanülenrohres (4) in konstant bleibendem Abstand vom Ansatz (6) vorgenommen wird.

3. Verfahren nach Anspruch 1 oder 2, dadurch gekennzeichnet, dass das Kanülenrohr (4) vor seinem Einspannen im Spannbacken (60) von einer Hilfskraft in seiner Ausgangslage festgelegt wird.

4. Verfahren nach Anspruch 3, bei dem das Kanülenrohr (4) beim Einbringen in den Spannbacken (60) gegen eine Prallfläche (72) geworfen wird, dadurch gekennzeichnet, dass das Kanülenrohr (4) von einer Rückführkraft an der Prallfläche (72) in Anlage gehalten wird, bis der Spannbacken (60) greift.

5. Verfahren nach Anspruch 3, dadurch gekennzeichnet, dass das Kanülenrohr (4) von einer Mitführungskraft an eine das Kanülenrohr im geöffneten Spannbacken (60) ausrichtenden Gegenfläche angelegt wird.

6. Verfahren nach einem der Ansprüche 1 bis 5, bei dem mehrere Einmalkanülen (2) zugleich hergestellt und dabei von einer gemeinsamen Widerstandsheizspannung beaufschlagt werden, dadurch gekennzeichnet, dass die Zahl der vom Spannbacken (60) erfassten Kanülenrohre (4) gezählt und die Widerstandsheizspannung und/oder die Schweissleistung und/oder die Schweisszeit entsprechend dem Zählergebnis eingestellt werden.

7. Verfahren nach einem der Ansprüche 1 bis 6, bei dem die Kanülenrohre (4) magaziniert und von einem Vereinzelungsschieber (42) entnommen werden, dadurch gekennzeichnet, dass eine am im Vereinzelungsschieber (42) liegenden Kanülenrohr (4) reibschlüssig einwirkende Entnahmekraft aufgebracht wird.

8. Verfahren nach einem der Ansprüche 1 bis 7, dadurch gekennzeichnet, dass ein im Ansatz (6) verlaufender Abschnitt des Kanülenrohrs (4) in reib- oder formschlüssigem Eingriff mit dem Ansatz aufgeweitet wird.

9. Verfahren nach Anspruch 8, dadurch gekennzeichnet, dass ein zug- und druckfester Eingriff hergestellt wird.

10. Verfahren nach Anspruch 8 oder 9, dadurch gekennzeichnet, dass eine Zugfestigkeit gegen eine Zugkraft von mehr als 3 kp, vorzugsweise mehr als 8 kp, hergestellt wird.

11. Verfahren nach einem der Ansprüche 8 bis 10, dadurch gekennzeichnet, dass ein Kanülenrohr (4) in eine vorgeformte Aussparung (174) im Ansatz (6) aufgeweitet wird.

12. Verfahren nach einem der Ansprüche 8 bis 11, dadurch gekennzeichnet, dass an der Aussenseite des Ansatzes (6) eine Materialaussparung (176, 178) vorgeformt wird, die bei dem Aufweiten des Kanülenrohres (4) durch verdrängtes Material des Ansatzes aufgefüllt wird.

13. Verfahren nach einem der Ansprüche 8 bis 12, dadurch gekennzeichnet, dass das Kanülenrohr (4) vor seinem Aufweiten auf der Schulter einer Einschnürung (152) einer axialen Bohrung (10) durch den Ansatz (6) aufgelagert wird, und dass zum Aufweiten ein Dorn (160) durch die Einschnürung hindurchgeführt wird.

14. Verfahren nach Anspruch 13, dadurch gekennzeichnet, dass das Kanülenrohr (4) vor dem Aufweiten auf der Schulter der Einschnürung (152) nur mit der Aussenzone seiner Wandstärke aufgelagert wird.

15. Verfahren nach einem der Ansprüche 1 bis 9, dadurch gekennzeichnet, dass die Gegenelektrode (98) an der Innenfläche des Kanülenrohres (4) angelegt wird.

16. Verfahren nach einem der Ansprüche 8 bis 15, dadurch gekennzeichnet, dass ein für das Aufweiten des Kanülenrohres (4) verwendeter Dorn (160) auch als Gegenelektrode verwendet wird.

17. Verfahren nach einem der Ansprüche 8 bis 16, dadurch gekennzeichnet, dass beim Aufweiten des Kanülenrohres (4) ein Aufweitdorn eingerüttelt oder eingedreht wird.

18. Vorrichtung zum Herstellen von Einmalkanülen (2) aus metallischen Kanülenrohren (4) und Ansätzen (6) aus Kunststoff mit einer Halteeinrichtung (26, 28) für den Ansatz, einer gegen das innere Ende (24) des Kanülenrohres anlegbaren Gegenelektrode (98) eines Schweissspannungsgenerators und einem am freien Ende (22) des Kanülenrohres angreifenden Spannbacken (60), dessen Abstand relativ zur Gegenelektrode veränderbar ist, gekennzeichnet durch eine am Umfang des Kanülenrohres (4) zwischen dem Aufnahmeort eines Ansatzes (6) in der Halteeinrichtung (26, 28) und dem Spannbacken (60) anlegbaren Elektrodenzange (100).

19. Vorrichtung nach Anspruch 18, bei welcher der Spannbacken (60) auf den Anordnungsort des Ansatzes (6) in der Halteeinrichtung (26, 28) zu mit Reibschluss gegenüber dem Kanülenrohr (4) verschiebbar ist, dadurch gekennzeichnet, dass die Elektrodenzange (100) eng benachbart der Austrittsstelle des Kanülenrohres (4) aus dem Kunststoffansatz (6) stationär und mit leichterem Reibschluss als der Spannbacken gegenüber dem Kanülenrohr angeordnet ist.

20. Vorrichtung nach Anspruch 18 oder 19, dadurch gekennzeichnet, dass die Elektrodenzange (100) in ihre Schliessstellung federvorgespannt (110) ist, und dass eine Öffnungsnockensteuerung (112, 114, 116, 118) vorgesehen ist.

21. Vorrichtung nach einem der Ansprüche 18 bis 20, dadurch gekennzeichnet, dass mindestens ein Backen der Elektrodenzange (100), vorzugsweise beide Backen, eine etwa punkt- oder linienförmige Kontaktfläche (108) hat.

22. Vorrichtung nach einem der Ansprüche 18 bis 20, dadurch gekennzeichnet, dass die Elektrodenzange (100) frei schwimmend gelagert (102) ist.

23. Vorrichtung nach einem der Ansprüche 18 bis 22, bei der eine Gleitstrecke für vereinzelte Kanülenrohre (4) an einer Prallfläche hinter dem geöffneten Spannbacken (60) mündet, gekennzeichnet durch eine das jeweilige Kanülenrohr (4) auf die Prallfläche (72) zu mitnehmende Friktionswalze (78).

24. Vorrichtung nach einem der Ansprüche 18 bis 22, in Mehrfachanordnung, gekennzeichnet durch eine Erfassungseinrichtung (122, 124) für die Anzahl der in den Spannbacken (60) gelagerten Kanülenrohre (4), einen Speicher (128) des erfassten Ergebnisses, einen Zähler (136) der in den Spannbacken gelagerten Kanülenrohre und eine Einstelleinrichtung (138) für die Schweissleistung in Abhängigkeit vom Zählergebnis.

25. Vorrichtung nach Anspruch 24, dadurch gekennzeichnet, dass die Erfassungseinrichtung (138) parallele Widerstandsmessstrecken (122) zwischen den an ein gemeinsames Potential gelegten Spannbacken (60) und den gegeneinander isolierten Anlagebereichen von den Spannbacken nachgeordneten Anlageflächen an dem Kanülenrohr (4), vorzugsweise den Prallflächen, (an 72), aufweist.

26. Vorrichtung nach einem der Ansprüche 18 bis 25, mit einem Kanülenrohrmagazin (30) mit Vereinzelungsschieber (42), gekennzeichnet durch eine im Vereinzelungsschieber (42) gelagerte, am Umfang des jeweils aufgenommenen Kanülenrohres (4) auswerfend angreifende Friktionswalze (50).

27. Vorrichtung nach Anspruch 26, dadurch gekennzeichnet, dass die Friktionswalze (50) im Vereinzelungsschieber (42) axial verschiebbar gelagert ist, und dass die Friktionswalze variablen Durchmesser hat, derart, dass der grössere Durchmesser (52) in Auswerfstellung des Vereinzelungsschiebers wirksam ist.

## Claims

1. A process for manufacturing disposable cannulae (2) comprising: melting a hub (6) of plastic against a metallic cannula tube (4) by inserting the cannula tube into the hub, clamping the cannula tube between a counter electrode (98) supporting the inner end (24) of the cannula tube and a clamping jaw (60) engaging at the free end (22) of the cannula tube as well as intermittently applying a resistance heating voltage between the counter electrode and the free end of the cannula tube protruding from the hub and simultaneously moving the clamping jaw and the counter electrode towards each other, characterized in that the resistance heating voltage is applied to the free end (22) of the cannula tube (4) between the hub (6) and the clamping jaw (60).

2. The process according to claim 1, in which, when applying the resistance heating voltage, the clamping jaw (60) is moved in the direction of the hub (6), characterized in that the applying of the resistance heating voltage to the free end (22) of the cannula tube (4) is effected at a constant distance from the hub (6).

3. The process according to claim 1 or claim 2, characterized in that the cannula tube (4), before being clamped in the clamping jaw (60), is fixed in its starting position by an assistant.

4. The process according to claim 3, in which the cannula tube (4) when inserted in the clamping jaw (60) is thrown against a baffle surface (72), characterized in that the cannula tube (4) is held in abutment to the baffle surface (72) by a restoring force until the clamping jaw (60) grips.

5. The process according to claim 3, characterized in that the cannula tube (4) is placed by an entraining force against a counter surface aligning the cannula tube in the opened clamping jaw (60).

6. The process according to any of claims 1 to 5, in which several disposable cannulae (2) are manufactured simultaneously while a common resistance heating voltage is applied to them, characterized in that the number of cannula tubes (4) grasped by the clamping jaws (60) is counted and the resistance heating voltage and/or the welding capacity and/or the welding time are adjusted according to the result of the count.

7. The process according to any of claims 1 to 6, in which the cannula tubes (4) are stored and removed by a singling and pushing device (42), characterized in that a removing force is applied, acting by friction contact, to the cannula tube (4) in the singling and pushing device (42).

8. The process according to any of claims 1 to 7, characterized in that a section of the cannula tube (4) in the hub (6) is expanded while in frictional and form-locked engagement with the hub.

9. The process according to claim 8, characterized in that a tension- and compression-proof engagement is produced.

10. The process according to claim 8 or claim 9, characterized in that a tensile strength is produced against a tensile load of more than 3 kp, preferably more than 8 kp.

11. The process according to any of claims 8 to 10, characterized in that a cannula tube (4) is expanded into a preformed recess (174) in the hub (6).

12. The process according to any of claims 8 to 11, characterized in that a recess in the material (176, 178) is preformed on the outside of the hub (6), said recess being filled by displaced material of the hub when expanding the cannula tube (4).

13. The process according to any of claims 8 to 12, characterized in that the cannula tube (4), before being expanded, rests on the shoulder of a constriction (152) of an axial bore (10) extending through the hub (6), and that for expansion a mandrel (160) is passed through said constriction.

14. The process according to claim 13, characterized in that the cannula tube (4), before being expanded, rests on the shoulder of the constriction (152) with the outer zone of its wall thickness only.

15. The process according to any of claims 1 to 9, characterized in that the counter electrode (98) is set to the inner surface of the cannula tube (4).

16. The process according to any of claims 8 to 15, characterized in that a mandrel (160) used for expanding the cannula tube (4) is also used as counter electrode.

17. The process according to any of claims 8 to 16, characterized in that when expanding the cannula tube (4) an expanding mandrel is shaken in or screwed in.

18. An apparatus for manufacturing disposable cannulae (2) from metallic cannula tubes (4) and hubs (6) of plastic, comprising a holding device (26, 28) for the hub, a counter electrode (98) of a welding voltage generator, which counter electrode may be set to the inner end (24) of the cannula tube, and a clamping jaw (60) engaging at the free end (22) of the cannula tube, the distance of said clamping jaw (60) relative to the counter electrode being variable, characterized by an electrode holder (100) which may be applied to the periphery of the cannula tube (4) between the receiving place for the hub (6) in the holding device (26, 28) and the clamping jaw (60).

19. The apparatus according to claim 18, in which the clamping jaw (60) can be moved with frictional engagement with respect to the cannula tube (4) towards the place where the hub (6) is positioned in the holding device (26, 28), characterized in that the position of the electrode holder (100) is stationary closely adjacent to the place where the cannula tube (4) comes out of the plastic hub (6) and that said electrode holder (100) has a lighter frictional engagement with respect to the cannula tube than the clamping jaw.

20. The apparatus according to claim 18 or claim 19, characterized in that the electrode holder (100) is spring-biassed (110) in its closing position and that a cam control (112, 114, 116, 118) is provided for opening.

21. The apparatus according to any of claims 18 to 20, characterized in that at least one jaw of the electrode holder (100), preferably both jaws, has/have a more or less point- or line-shaped contact area (108).

22. The apparatus according to any of claims 18 to 20, characterized in that the electrode holder (100) is mounted so as to float freely (102).

23. The apparatus according to any of claims 18 to 22, in which a sliding section for singled cannula tubes (4) ends at a baffle surface behind the opened clamping jaw (60), characterized by a friction roller (78) taking the respective cannula tube (4) towards the baffle surface (72).

24. The apparatus according to any of claims 18 to 22, in multiple arrangement, characterized by a detecting device (122, 124) for the number of the cannula tubes (4) in the clamping jaws (60), a memory (128) of the detected result, a counter (136) of the cannula tubes in the clamping jaws and an adjusting device (138) for the welding capacity depending on the result of the count.

25. The apparatus according to claim 24, characterized in that the detecting device (138) has parallel resistance measuring sections (122) at the cannula tubes (4) between the clamping jaws (60) applied to a common potential and the mutually insulated abutment portions of abutment surfaces arranged subsequent to the clamping jaws, preferably the baffle surfaces (at 72).

26. The apparatus according to any of claims 18 to 25, comprising a cannula tube store (30) with singling and pushing device (42), characterized by a friction roller (50) mounted in the singling and pushing device (42) and engaging at the periphery of each cannula tube (4) received and thereby ejecting it.

27. The apparatus according to claim 26, characterized in that the friction roller (50) is mounted to permit axial movement in the singling and pushing device (42) and that the friction roller has a variable diameter in such a way that the larger diameter (52) is effective in the ejecting position of the singling and pushing device.

**Revendications**

1. Procédé pour fabriquer des canules (2) à usage unique, comprenant la fixation d'une canule proprement dite ou aiguille creuse métallique (4) avec fusion dans un embout (6) en plastique, par introduction de l'aiguille dans l'embout, serrage de l'aiguille entre une contre-électrode (98) formant butée pour l'extrémité intérieure (24) de l'aiguille et une griffe de serrage (60) tenant la partie libre (22) de l'aiguille, ainsi qu'application temporaire d'une tension électrique de chauffage par effet Joule entre la contre-électrode et la partie libre de l'aiguille, dépassant de l'embout, et approche mutuelle simultanée de la griffe et de la contre-électrode, caractérisé en ce que la tension de chauffage est appliquée à la partie libre (22) de l'aiguille (4) entre l'embout (6) et la griffe (60).

2. Procédé selon la revendication 1, dans lequel la griffe (60) est déplacée en direction de l'embout (6) pendant l'application de la tension de chauffage, caractérisé en ce que la tension de chauffage est appliquée à la partie libre (22) de l'aiguille à une distance de l'embout (6) restant constante.

3. Procédé selon la revendication 1 ou 2, caractérisé en ce qu'une force auxiliaire immobilise l'aiguille (4) à une position de départ avant son serrage dans la griffe (60).

4. Procédé selon la revendication 3, dans lequel l'aiguille (4) est projetée contre une surface de rebondissement ou d'arrêt (72) en vue de sa préhension par la griffe (60), caractérisé en ce qu'une force de rappel maintient l'aiguille (4) en appui contre la surface d'arrêt (72) jusqu'à la fermeture de la griffe (60) sur elle.

5. Procédé selon la revendication 3, caractérisé en ce qu'une force d'entraînement applique l'aiguille (4) contre une surface d'appui qui positionne l'aiguille dans la grille (60) ouverte.

6. Procédé selon l'une des revendications 1 à 5, dans lequel plusieurs canules (2) à usage unique sont fabriquées en même temps et une tension commune de chauffage par effet Joule est appliquée à elles pendant la fabrication, caractérisé en ce qu'il comprend le comptage des aiguilles (4) saisies par la griffe (60) et le réglage, suivant le résultat de ce comptage, de la tension de chauffage par effet Joule et/ou de la puissance de soudage et/ou du temps de soudage.

7. Procédé selon l'une des revendications 1 à 6, dans lequel les aiguilles (4) sont magasinées et sont enlevées du magasin par un poussoir d'alimentation sérielle (42), caractérisé par la production d'une force d'enlèvement agissant par frottement sur l'aiguille (4) disposée dans le poussoir d'alimentation (42).

8. Procédé selon l'une des revendications 1 à 7, caractérisé en ce qu'il comprend l'élargissement d'un tronçon d'aiguille (4) situé dans l'embout (6) et relié à lui par frottement ou par sûreté de forme.

9. Procédé selon la revendication 8, caractérisé en ce que la liaison établie entre l'aiguille et l'embout est une liaison qui résiste à la traction et à la compression.

10. Procédé selon la revendication 8 ou 9, caractérisé en ce que la liaison entre l'aiguille et l'embout résiste à une traction de plus de 3 kg, de préférence de plus de 8 kg.

11. Procédé selon l'une des revendications 8 à 10, caractérisé en ce que l'aiguille (4) est élargie dans un évidement préformé (174) de l'embout (6).

12. Procédé selon l'une des revendications 8 à 11, caractérisé en ce que l'embout (6) présente extérieurement un amincissement préformé (176, 178) qui est rempli par du matériau refoulé de l'embout lors de l'élargissement de l'aiguille (4).

13. Procédé selon l'une des revendications 8 à 12, caractérisé en ce que l'aiguille (4), avant son élargissement, est disposée en appui contre un épaulement formé par un rétrécissement (152) dans l'alésage axial (10) de l'embout (6) et en ce que, pour l'élargissement, un mandrin (160) est passé à travers le rétrécissement.

14. Procédé selon la revendication 13, caractérisé en ce que, avant l'élargissement, l'aiguille (4) est seulement appuyée par la zone extérieure de son épaisseur de paroi contre l'épaulement formé par le rétrécissement (152).

15. Procédé selon l'une des revendications 1 à 9, caractérisé en ce que la contre-électrode (98) est appliquée contre la surface intérieure de l'aiguille.

16. Procédé selon l'une des revendications 8 à 15, caractérisé en ce que le mandrin (160) utilisé pour l'élargissement de l'aiguille (4) est employé en même temps comme contre-électrode.

17. Procédé selon l'une des revendications 8 à 16, caractérisé en ce que, pour l'élargissement de l'aiguille (4), on utilise un mandrin qui est enfoncé dans l'aiguille pendant qu'il est animé d'une vibration ou d'une rotation.

18. Appareil pour fabriquer des canules (2) à usage unique constituées d'une canule proprement dite ou aiguille creuse métallique (4) et d'un embout en plastique (6), comprenant un dispositif de maintien (26, 28) pour l'embout, une contre-électrode (98) reliée à un générateur de tension électrique de soudage et susceptible d'être appliquée contre l'extrémité intérieure (24) de l'aiguille, ainsi qu'une griffe de serrage (60) qui saisit l'aiguille par sa partie libre (22) et dont la distance par rapport à la contre-électrode est variable, caractérisé en ce qu'il comprend une pince-électrode (100) applicable autour de l'aiguille (4) entre le point de réception de l'embout (6) dans le dispositif de maintien (26, 28) et la griffe (60).

19. Appareil selon la revendication 18, dans lequel la griffe (60) est déplaçable, en frottant sur l'aiguille (4), en direction du point de réception de l'embout (6) dans le dispositif de maintien (26, 28), caractérisé en ce que la pince-électrode (100) est disposée fixe à proximité du point de sortie de l'aiguille (4) de l'embout (6) et de manière que son frottement sur l'aiguille soit plus faible que le frottement de la griffe sur l'aiguille.

20. Appareil selon la revendication 18 ou 19, caractérisé en ce que la pince-électrode (100) est précontrainte élastiquement (110) dans le sens de sa fermeture et en ce que l'appareil comprend une commande d'ouverture à came (112, 114, 116, 118).

21. Appareil selon l'une des revendications 18 à 20, caractérisé en ce que l'une au moins des mâchoires de la pince-électrode (100), de préférence les deux, présente(nt) une surface de contact à peu près ponctuelle ou linéaire (108) avec l'aiguille.

22. Appareil selon l'une des revendications 18 à 20, caractérisé en ce que la pince-électrode (100) est montée librement flottante (102).

23. Appareil selon l'une des revendications 18 à 22, dans lequel un parcours de glissement pour aiguilles (4) séparées se termine à une surface de rebondissement ou d'arrêt située derrière la griffe (60) ouverte, caractérisé en ce qu'il comprend un rouleau de friction (78) pour entraîner les aiguilles successives jusque contre la surface d'arrêt (72).

24. Appareil selon l'une des revendications 18 à 22, pour la fabrication simultanée de plusieurs canules, caractérisé en ce qu'il comprend un dispositif de saisie de données (122, 124) pour déterminer le nombre d'aiguilles (4) disposées dans la griffe (60), une mémoire (128) pour le résultat de la saisie, un compteur (136) pour les aiguilles disposées dans la griffe et un dispositif (138) pour régler la puissance de soudage en fonction de la valeur indiquée par le compteur.

25. Appareil selon la revendication 24, caractérisé en ce que le dispositif de saisie de données (138) possède des circuits de mesure de résistance parallèles (122) entre les griffes (60), appliquées à un potentiel commun, et des zones mutuellement isolées situées à la suite des griffes et contre lesquelles viennent s'appliquer les aiguilles (4), de préférence des zones de contact d'une surface de rebondissement ou d'arrêt (72).

26. Appareil selon l'une des revendications 18 à 25, comportant un magasin à aiguilles (30) combiné avec un poussoir d'alimentation sérielle (42), caractérisé en ce qu'il comprend un rouleau de friction (50) monté dans le poussoir d'alimentation (42) et agissant sur le pourtour de l'aiguille (4) reçue dans le poussoir en vue de son éjection de celui-ci.

27. Appareil selon la revendication 26, caractérisé en ce que le rouleau de friction (50) est monté axialement mobile dans le poussoir d'alimentation (42) et en ce que le rouleau de friction possède un diamètre variable, l'agencement étant tel que le plus grand diamètre (52) agit sur l'aiguille en position d'éjection du poussoir.

**Fig. 1**

Fig. 2

Fig. 3

Fig. 4

**Fig. 5**

**Fig. 6**

0 030 353

**_Fig. 7_**

**_Fig. 8a_**   **_Fig. 8b_**

**_Fig. 9a_**   **_Fig. 9b_**

27

4

6

10

10a

10b

200

201

200

10b

6

201

6

200

Fig. 13   Fig. 14   Fig. 15

0 030 353

Fig. 16          Fig. 17          Fig. 18

0 030 353